# EUROPÄISCHE PATENTSCHRIFT

(11) **EP 0 892 237 B1**
(45) Veröffentlichungstag und Bekanntmachung des Hinweises auf die Patenterteilung: **29.01.2003**
(21) Anmeldenummer: 98113916.5
(22) Anmeldetag: 06.10.1994
(51) Int. Cl.: F41A 3/54, F41A 3/26, F41A 9/32

(54) **Selbstlade-Granatwerfer**
Self-loading gun
Arme à feu automatique

(30) Priorität: 08.10.1993 DE 4334412
(43) Veröffentlichungstag der Anmeldung: 20.01.1999
(62) Teilanmeldung aus: 94929517.4
(73) Patentinhaber: HECKLER & KOCH GMBH, D-78727 Oberndorf (DE)
(72) Erfinder: Weichert, Berthold, 78658 Zimmern (DE); Wössner, Ernst, 72172 Sulz (DE); Gielke, Gerhard, 78727 Oberndorf (DE); Gablowski, Jürgen, 78727 Oberndorf (DE)
(74) Vertreter: von Samson-Himmelstjerna, Friedrich R., Dipl.-Phys.

(56) Entgegenhaltungen:
- EP-A- 0 378 976
- DE-A- 2 016 281
- DE-C- 700 629
- GB-A- 629 224
- US-A- 2 960 917
- US-A- 4 942 802

## Beschreibung

Die Erfindung betrifft einen Selbstlade-Granatwerfer mit den Merkmalen des Oberbegriffs des Anspruchs 1.

Ein solcher Granatwerfer ist in der Zeitschrift "International Defense Review", Band 22, Nr, 12/1989 beschrieben. Er weist eine Patronengurt-Zuführeinrichtung auf, die die jeweils vorderste Patrone unmittelbar hinter das Patronenlager fördert. Ein Masseverschluß, der in der Wirkungsweise dem einer Maschinenpistole ähnelt, wird durch eine Schließfederanordnung gegen diese vorderste Patrone gefördert, schiebt sie in das Patronenlager und zündet sie.

Ferner ist ein Zubringerdeckel für ein Maschinengewehr bekannt (DE-C-700 619), der die Patronengurt-Zuführeinrichtung mit einem Kurvenhebel umfaßt, der am hinteren Ende des Deckels schwenkbar gelagert ist und in den der Verschluß eingreifen kann.

Im folgenden werden Begriffe wie "vorne", "hinten", "seitlich" usw. ohne nähere Definition verwendet. Sie beziehen sich stets auf die in horizontaler Feuerstellung befindliche Waffe, wobei "vorne" die Mündung, also das in Schußrichtung vorderste Ende der Waffe, bedeutet.

Um den Granatwerfer feuerbereit zu machen, genügt es, den Masseverschluß gegen die Wirkung der Schließfederanordnung in seine hinterste Lage zu bewegen, in welcher er von einer Abzugseinrichtung festgehalten wird, und den Patronengurt in die Zuführeinrichtung einzulegen.

In diesem einfachen Bewegungsablauf liegt der besondere Vorteil des gattungsbildenden Granatwerfers gegenüber dem bisher am weitesten verbreiteten Selbstlade-Granatwerfer, dem US Mark 19: bei diesem muß nämlich nach dem Einlegen des Gurtes der Verschluß zunächst einmal leer abgeschlagen und dann wieder gespannt werden, da die vorderste Patrone des Gurtes beim ersten Abschlagen des Verschlusses nicht unmittelbar in das Patronenlager, sondern zunächst in eine Übergabeposition gefördert wird, aus welcher sie durch das zweite Abschlagen des Verschlusses in das Patronenlager gefördert und dort gezündet wird.

Dieser komplizierte Ladevorgang führt zu Bedienungsfehlern, aufgrund deren die Waffe nach dem Laden entweder nicht feuerbereit ist oder unzeitig abgefeuert wird.

Der gattungsbildende Granatwerfer, der diesen Nachteil nicht aufweist und baulich recht einfach ist, hat allerdings eine unbefriedigende Betriebs- und Funktionssicherheit.

Ausgehend von diesem Stand der Technik liegt der Erfindung die Aufgabe zugrunde, einen Selbstlade-Granatwerfer zu schaffen, der unter Nutzung der oben beschriebenen Vorzüge des gattungsbildenden Granatwerfers eine bessere Betriebssicherheit, eine bessere Funktionssicherheit, eine bessere Bedienbarkeit, einen einfacheren Aufbau und/oder einen kostengünstigeren Aufbau aufweist.

Diese Aufgabe wird durch die Merkmale des Anspruchs 1 gelöst.

Beim eingangs genannten, gattungsbildenden, bekannten Granatwerfer erfolgt die Steuerung der Schieber der Patronengurt-Zuführeinrichtung über Nuten, die im Masseverschluß ausgebildet sind und in denen Mitnehmer laufen, die auf den Schiebern sitzen.

Diese an sich sehr einfache Steuerung hat sich allerdings als unbefriedigend erwiesen. Der Grund hierfür dürfte in der Tatsache liegen, daß sich die Schieber während sehr kurzer Bewegungsstrecken des Masseverschlusses über verhältnismäßig lange Strecken bewegen müssen, so daß ihre unmittelbare Führung über stark abgewinkelte Führungsnuten erfolgen muß, damit zu hohen Kräften führt und etwa bei Verschmutzung nicht mehr zuverlässig erfolgen kann. Außerdem ist der Verschleiß hoch.

Erfindungsgemäß wird dieses Problem mangelnder Funktionssicherheit und Verschleißbeständigkeit durch die Merkmale des Anspruchs 1 gelöst.

Hierbei ist ein gesonderter Kurvenhebel vorgesehen, der sich längs der Bewegungsbahn des Masseverschlusses erstreckt und mit diesem über eine aus Führungskurve und Mitnehmer gebildete Steuerverbindung gesteuert wird. Der Kurvenhebel ist mit den Schiebern über ein Gestänge verbunden.

Somit ist es möglich, günstige Kräfteverhältnisse im Eingriff zwischen Führungskurve und Mitnehmer herzustellen. Außerdem ist es möglich, das Material des Kurvenhebels, der bevorzugt eine Führungsnut aufweist, hinsichtlich besonders geringen Verschleißes zu optimieren. Auch der Mitnehmer, der vorzugsweise aus einer am Masseverschluß angebrachten Rolle besteht, kann optimiert werden, wobei sein Gewicht zu dem ohnehin erforderlichen Gewicht des Masseverschlusses beiträgt und somit unproblematisch ist.

Gemäß Anspruch 2 ist der Kurvenhebel an seinem vorderen Ende schwenkbar gelagert; seine Schwenkbewegung wird hinter seiner Mitte von einem zweiarmigen Umlenkhebel abgegriffen und nach vorne übertragen. Diese auf den ersten Blick umständlich erscheinende Anordnung ermöglicht eine Kraftübertragung vom Kurvenhebel auf die Schieber ohne Übersetzung. Die notwendigen Toleranzen zwischen Mitnehmer und Steuerkurve, die die zuverlässige Funktion der Waffe auch bei Verschmutzung sicherstellen, werden somit ebenfalls nicht übersetzt.

Gemäß Anspruch 3 ist die Hebelsteuerung, die das Gestänge mit den Schiebern verbindet, mittig angeordnet und mit zwei Steuerhebeln symmetrisch ausgebildet. Diese Anordnung ist eine Vorbedingung für die Austauschbarkeit der Schieber und damit für die freie Wahl der Richtung der Zuführung des Patonengurtes.

Der Anspruch 4 befaßt sich mit der näheren Ausbildung der Steuerhebel, die in einem aufklappbaren Deckel gelagert sind und erfindungsgemäß mit einer lösbaren Kupplung versehen sind, mit welcher sie problemlos in und außer Eingriff mit dem Gestänge gelangen, das im Gehäuse der Waffe oberhalb des Masseverschlusses sitzt. Hierbei erfolgt die Steuerung einfacherweise nur über einen der beiden Steuerhebel, der sie seinerseits auf den anderen Steuerhebel überträgt.

Diese Übertragung erfolgt gemäß Anspruch 5 durch eine gelenkige Verbindung zwischen Schenkeln der Steuerhebel. Diese gelenkige Verbindung ist gemäß Anspruch 6 ohne Verwendung irgendeines Zwischenelementes durchgeführt.

Der Eingriff eines jeden Steuerhebels in den ihm zugeordneten Schieber erfolgt gemäß Anspruch 7 in jeweils gleichartiger Weise, was nicht nur eine Herstellungsvereinfachung ermöglicht, sondern eine weitere Vorbedingung für die oben bereits angesprochene Austauschbarkeit der Schieber ist.

Gemäß Anspruch 8 ist jeder Schieber in einer Querführung geführt; diese beide Querführungen sind gleichartig so ausgebildet, daß die beiden Schieber austauschbar sind.

Bei diesem Austausch werden die Schieber nicht nur ausgetauscht, sondern in ihrer Richtung umgekehrt, also umgedreht, so daß die Querführungen zu ihrer Längsachse symmetrisch ausgebildet oder wirksam sein müssen.

Dieser Austausch der Schieber ermöglicht es, die Waffe je nach Bedarf mit der Zuführung von rechts oder links auszubilden, so daß beim Einbau der Waffe etwa im Einstieg eines Hubschraubers oder an der Luke eines Panzerfahrzeuges die Zuführung des Patronengurtes von der optimal geeigneten Seite her erfolgen kann.

Es ist eine weitere Aufgabe der Erfindung, diese Zuführung so auszubilden, daß sie zu diesem Zweck geeignet ist und außerdem mit größter Funktionssicherheit den Patronengurt nachführt und entgurtet.

Diese Aufgabe wird durch die Merkmale des Anspruchs 9 gelöst.

Die einzelnen Patronen sind etwa in der Längsmitte der Patronenhülse von einem wie eine Rohrschelle ausgebildeten Gurtglied reibschlüssig umgriffen, das auf der einen Seite einen gelenkig am Gurtglied befestigten, mit einem Kopf versehenen Zapfen und auf der gegenüberliegenden Seite einen abstehenden, bügelförmigen Abschnitt mit einem Langloch aufweist. Das Langloch ist am vorderen Ende erweitert und so bemessen, daß der Kopf des Zapfens der benachbarten Patrone die Erweiterung des Langloches, aber nicht dessen übrigen Abschnitt durchsetzen kann, in dem der Hals des Zapfens geführt ist. Mittels der Zapfen-Langloch-Verbindung sind die einzelnen Gurtglieder gelenkig aneinandergehängt.

Bei der Schieberanordnung, die in Anspruch 9 beschrieben ist, bewegen sich beim Vorlauf des Masseverschlusses beide Schieber gegenläufig jeweils von außen aus einer Ausgangslage nach innen, wobei die Innenklinke des ersten Schiebers die erste Patrone hintergreift und bis vor das Patronenlager fördert. Gleichzeitig bewegt sich der zweite Schieber aus seiner Ausgangslage gegenläufig und gelangt mit der an ihm angebrachten festen Stütze bis neben die erste Patrone, wo diese Stütze verhindert, daß die Patrone über ihre Lage hinter dem Patronenlager hinausrutscht. Nun ist der Masseverschluß hinter dieser Patrone angelangt und schiebt sie in das Patronenlager ein, wobei das Gurtglied auf dem Rand des Patronenlagers aufsitzt und auf der Patrone nach hinten rutscht. Bei der Zündung der Patrone befinden sich das Gurtglied und der hintere, von ihm umschlossene Teil der Patronenhülse außerhalb des Patronenlagers.

Beim Rücklauf schiebt die abgefeuerte Patronenhülse den Masseverschluß nach hinten bis zu einer Position hinter der Patronengurt-Zuführeinrichtung, wo die Patronenhülse auf einen üblichen Ausstoßer auftrifft und durch ein einziges, seitliches Fenster ausgeworfen wird; gleichgültig, ob die Zuführung des Patronengurtes von rechts oder links erfolgt.

Gleichzeitig bewegen sich die beiden Schieber in ihre jeweilige Ausgangslage zurück, wobei die Innenklinke des ersten Schiebers über die nachfolgende Patrone hinwegschwenkt und die Stütze des zweiten Schiebers sich nach außen bewegt, um das Ausziehen der Patronenhülse nicht zu stören. Gleichzeitig schiebt die an diesem zweiten Schieber angeordnete Schwenkklinke die nachfolgende Patrone weiter in eine Position, in welcher sie von der genannten Innenklinke beim nächsten Vorlauf des Masseverschlusses weitergeführt wird.

Wie ersichtlich, erfolgt die Nachführung einer jeden Patrone in zwei aufeinanderfolgenden Schritten jeweils beim Vorlauf und Rücklauf des Masseverschlusses, so daß zu hohe Beschleunigungen und somit Massekräfte vermieden sind.

Es wird ausdrücklich darauf hingewiesen, daß hier und in den Ansprüchen jeweils nur von einer Klinke, Stütze usw. die Rede ist, aber bevorzugt mehrere, am besten zwei, derartige Elemente in Längsrichtung der Waffe (quer zum Patronengurt) nebeneinanderliegend angeordnet sind, um die Patrone bei allen ihren Bewegungen stets zur Längsrichtung der Waffe ausgerichtet zu halten.

Somit ist die Waffe geeignet, auch einen Patronengurt der genannten Art störungsfrei aufzunehmen, bei dem infolge der Befestigung der Gurtglieder untereinander diese gegeneinander verschwenkbar sind.

Gemäß Anspruch 10 sitzt am innenliegenden Ende des ersten Schiebers ein fester Anschlag ähnlich der Stütze am Außenende des zweiten Schiebers; diese Stütze hat die Aufgabe, zu verhindern, daß der bei offenem Verschluß nachgeführte Patronengurt mit seiner dann vordersten Patrone über die Position hinausrutscht, die diese dann einnehmen soll, wenn sie von der Innenklinke erfaßt und nachgeführt werden soll.

Es hat sich bei Versuchen herausgestellt, daß der Patronengurt beim Feuern sehr heftige, peitschenartige Bewegungen ausführt und Erschütterungen erfährt, die das Einführen der hinter das Patronenlager nachgeführten, auf dieses ausgerichteten, vordersten Patrone beeinträchtigen kann.

Um diesem Nachteil entgegenzuwirken, wird erfindungsgemäß vorgeschlagen, unmittelbar vor dem bzw. während des Einführens der Patrone in das Patronenlager den Patronengurt, von dem diese Patrone bereits abgetrennt bzw. ausgegurtet ist, von dieser Patrone ein wenig zurückzuziehen; der Patronengurt kann nun nicht mehr durch seine unvermeidlichen Bewegungen gegen die Patrone anschlagen, die gerade in das Patronenlager eingeführt wird.

Es ist gleichzeitig auch von Vorteil, die am zweiten Schieber angebrachte Stütze von der Patrone weg zurückzufahren, damit sie nicht vom Kopf des Masseverschlusses gewaltsam zur Seite gedrückt wird und dabei unnötig hohem Verschleiß ausgesetzt ist.

Während man den genannten Störungen bisher dadurch begegnete, daß man die Patrone mit großem seitlichem Spiel vor dem Patronenlager anordnete, wird bei der Erfindung die Patrone exakt vor dem Patronenlager gehalten, bis der Einführvorgang einsetzt, und erst dann von ihren seitlichen Führungen freigegeben. Im Ergebnis erzielt die Erfindung somit eine sehr hohe Funktionssicherheit, ungeachtet der Lage und Ausrichtung der Waffe oder der auf diese einwirkenden Beschleunigungen. Die erfindungsgemäße Waffe kann somit auch auf einem durch das Gelände fahrenden Fahrzeug während der Fahrt geschossen werden, ohne daß Fahrbahnstöße die Nachladefunktion beeinträchtigen.

Gemäß der bevorzugten Ausgestaltung nach Anspruch 11 wird diese Freigabe der Patrone dadurch bewirkt, daß die Bewegungsumkehr der Schieber nicht erst beim Abschuß, also in vorderster Stellung des Masseverschlusses, sondern schon ein wenig früher einsetzt, so daß eine_am ersten Schieber angebrachte weitere Außenklinke, die zu diesem Zeitpunkt gegen Abkippen gesperrt ist, den Patronengurt zurückzieht und sich die Stütze von der gerade in das Patronenlager eingeführten Patrone wegbewegt.

Um diese Außenklinke zu sperren, also um ihr Abkippen zu verhindern, ist gemäß Anspruch 12 der zweite Schieber so ausgebildet, daß er die Außenklinke übergreift und somit sperrt. Somit gelingt es, diese Sperrfunktion ohne zusätzliches Bauteil in genauer Zuordnung zum Bewegungsablauf der beiden Schieber zu realisieren.

Wie bereits oben erläutert, befindet sich die vorderste Patrone des Patronengurtes in einer Zwischenposition, wenn sich der Masseverschluß in seiner hintersten Lage (Auslöselage) befindet. In dieser Zwischenposition wird die vorderste Patrone von der Schwenkklinke des zweiten Schiebers hintergriffen und abgestützt.

Beim Einlegen des Patronengurtes ist aber der diese Schwenkklinke tragende Deckel hochgeklappt.

Außerdem liegt in der Feuerbereitschaft die vorderste Patrone ständig gegen diese Schwenkklinke an, so daß sie allen vom Patronengurt ausgeübten Massenkräften standhalten muß.

Um die genaue Lage des Patronengurtes bei dessen Einlegen und bei offenem Deckel zu fixieren, und um die Massekräfte des Patronengurtes aufzunehmen, ist gemäß Anspruch 14 unterhalb des zugeführten Patronengurtes ein wie eine Klinke wirksamer, nach oben abgefederter Sperrhebel angeordnet, gegen dessen zur Längsmitte der Waffe hin weisendes, abstehendes Ende die vorderste Patrone mit ihrer Außenseite in der genannten Zwischenposition anliegt.

Wird der Gurt weitergefördert, dann weicht der Sperrhebel der nachfolgenden Patrone nach unten aus, ohne sie zu behindern, und taucht dann, wenn sie sich in der Zwischenlage - befindet, wieder nach oben, um wieder als Abstützung wirksam zu sein. Somit wird nicht nur ein Anschlag zum präzisen Einlegen des Patronengurtes geschaffen, sondern auch das.Ausleiern oder gar Abbrechen der Schwenkklinke oder mit ihr verbundener Bauteile verhindert.

Die für den erfindungsgemäßen Granatwerfer verwendete Patrone ist eine Randpatrone, also eine Patrone mit einem radial überstehenden Rand. Außerdem trägt diese Patrone ein Gurtglied. Liegt eine solche Patrone auf einer ebenen Fläche, dann ist die Längsachse der Patrone zu dieser Fläche geneigt. Das Bewegen einer solchen Patrone exakt parallel zu ihrer Längsachse ist somit problematisch.

Angesichts dieser Problemlage zielt die Erfindung darauf ab, das exakte und damit besonders störungsfreie Einführen der Patrone in das Patronenlager zu ermöglichen.

Diese Aufgabe wird durch die Merkmale des Anspruchs 23 gelöst.

Hierbei ist unterhalb des Patronenlagers ein Führungstisch ausgebildet, auf dem der herangeförderte Patronengurt aufliegt und glatt und störungsfrei bis auf eine Patronenauflage nachgefördert werden kann.

Wenn der Ausgurtvorgang erfolgt und die vorderste Patrone in das Patronenlager eingeführt wird, dann weicht die Patronenauflage so nach unten aus, daß sich die an ihrem Boden vom Kopf des Masseverschlusses erfaßte Patrone auf diesen ausrichten kann und somit einwandfrei in das Patronenlager eingeführt wird.

Die Patronenspitze verbleibt dabei stets auf der Höhe der Mitte des Patronenlagers; lediglich der hintere Teil taucht beim Ausweichen der Patronenauflage soweit ab, daß die Achse der Patrone voll auf die Seelenachse ausgerichtet ist.

Um sicherzustellen, daß die Patronenauflage nicht zur Unzeit nachgibt, ist sie gemäß Anspruch 24 durch die Bewegung des Masseverschlusses gesteuert und wird von diesem erst dann ausgelöst, kurz bevor oder wenn er die vorderste Patrone ausgurtet und dabei in das Patronenlager einführt.

Die Patronenauflage trägt die verhältnismäßig schwere Patrone mindestens für eine kurze Zeit, wobei auf die Waffe einwirkende Stöße die Belastung der Patronenauflage vervielfachen können. Eine Federbelastung allein ist daher wohl nicht ausreichend, um sicherzustellen, daß die Patronenauflage erst dann nachgibt, wenn sie vom Masseverschluß angesteuert wird.

Um diesem Problem abzuhelfen, ist gemäß Anspruch 25 eine Arretierung vorgesehen, die die Patronenauflage in ihrer normalen Lage festhält. Die Arretierung wird vom Masseverschluß gelöst, so daß das Lösen der Arretierung stets zuverlässig auf das Absenken der Patronenauflage, das ebenfalls vom Masseverschluß gesteuert ist, abgestimmt ist.

Die Patronenauflage kann als Platte ausgebildet werden, ist aber gemäß Anspruch 26 oder 27 als Anordnung aus mindestens einem Querfinger ausgebildet, dessen Masse verhältnismäßig gering ist, so daß sein rasches Ausweichen und Zurückschwenken keine Störungen in der Verschlußbewegung und insbesondere keinen hohen Verschleiß erbringt.

Wie bereits erwähnt, trachtet der Patronengurt, der bei Dauerfeuer ruckartig nachgeführt wird, dazu, heftige, peitschenartige Bewegungen durchzuführen, die zu Funktionsstörungen führen können.

Um diese Bewegungen abzumildern und für einen glatten Einlauf des Patronengurtes in die Waffe zu sorgen, ist gemäß Anspruch 15 eine den Patronengurt von unten her stützende Gurtführungsbühne und gemäß Anspruch 16 eine den Patronengurt von oben her führende Abdeckung vorgesehen, die beide lösbar am Gehäuse der Waffe anbringbar sind. Während die Gurtführungsbühne und die Abdeckung den Patronengurt unten bzw. oben an die Patronengurt-Einlauföffnung des Waffengehäuses angrenzen, ist diese Öffnung an ihrem vorderen und hinteren Ende gemäß Anspruch 17 je durch eine frei drehbare Patronengurt-Führungsrolle begrenzt. Beide Patronengurt-Führungsrollen weisen einen solchen Durchmesser auf, daß ein Verhaken der Patronen mit den Rollen unmöglich ist. Vorzugsweise sind beide Patronengurt-Führungsrollen kreiszylindrisch und weisen den gleichen Durchmesser auf.

Wie bereits oben mehrfach erwähnt, kann die erfindungsgemäße Einrichtung zum Zuführen und Entgurten des Patronengurtes für die Zuführung von rechts oder von links wahlweise und bevorzugt ohne Verwendung von irgendwelchen Austauschteilen umgestellt werden. Dementsprechend weist das Waffengehäuse gemäß Anspruch 18 zwei einander gegenüberliegende Einführungsöffnungen für den Patronengurt auf.

Diese Einführungsöffnungen sind bevorzugt an beiden Seiten des Gehäuses angeordnet, können aber bei besonderer Verwendung des Granatwerfers etwa in Land-, See- oder Luftfahrzeugen auch an der Ober- und Unterseite des Gehäuses angeordnet sein.

Um eine unnötige Verschmutzung zu vermeiden, ist gemäß Anspruch 19 die gerade nicht benutzte Einführungsöffnung durch eine Wandung oder Abdeckung verschließbar, die bevorzugt als anmontierbare Blechplatte ausgebildet ist, aber auch als einklemmbarer Kunststoffstopfen ausgebildet sein kann.

Die obengenannte Gurtführungsbühne und Abdeckung sind zu ihrer Mittelachse, die jeweils quer zur Waffenlängsachse verläuft, symmetrisch ausgebildet, so daß sie vor jeder der Einführungsöffnungen angebracht werden können.

Um die störungsfreie Zuführung des in einem Patronenkasten verwahrten Patronengurtes noch weiter zu verbessern, sind an diesem Patronenkasten und am Waffengehäuse komplementäre Halterungsmittel ausgebildet, die eine Halterung nahe der oder jeder Einführungsöffnung am Gehäuse umfaßt (Anspruch 20).

Die Abdeckung kann ein ständig am Waffengehäuse angebrachtes Bauelement sein, bildet gemäß Anspruch 21 jedoch bevorzugt einen Teil des Patronenkastens, so daß die Abdeckung, nachdem sie am Waffengehäuse angebracht ist, eine durchgehende, zuverlässige Führung für den Patronengurt vom Patronenkasten in die Waffe bildet.

Bevorzugt ist die Abdeckung am Deckel des Patronenkastens angebracht oder bildet mit diesem ein Teil (Anspruch 22).

Der Gegenstand der Erfindung wird anhand der beigefügten, schematischen Zeichnung beispielsweise noch näher erläutert, in der eine einzige bevorzugte Ausführungsform des erfindungsgemäßen Granatwerfers gezeigt ist. In dieser Ausführungsform sind alle oben aufgeführten Merkmale der Ansprüche vereinigt.

In der Zeichnung zeigt:
- Fig. 1: den Längs-Aufriß eines Ausführungsbeispiels eines erfindungsgemäßen Granatwerfers, mit abgenommenem Gehäusedeckel und Zubringerdeckel,
- Fig. 2: eine Draufsicht auf den in Fig. 1 gezeigten Granatwerfer,
- Fig. 3: einen schematischen Schnitt durch den Granatwerfer längs Linie III - III in Fig. 1,
- Fig. 4: einen Schnitt ähnlich dem der Fig. 3, wobei die Zubringerstellung nach Einlegen des Patronengurtes dargestellt ist, wobei sich der Masseverschluß in seiner Auslöselage (hintersten Lage) befindet,
- Fig. 5: einen Schnitt wie in Fig. 4, nachdem der Masseverschluß seine Vorwärtsbewegung begonnen hat,
- Fig. 6: einen Schnitt wie in Fig. 4, wobei sich die vorderste Patrone in Zuführposition befindet,

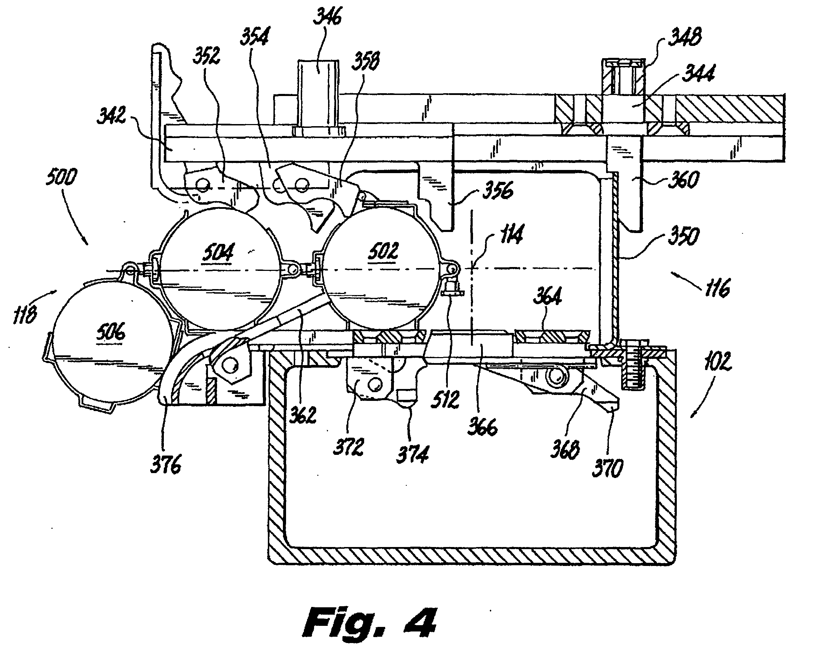

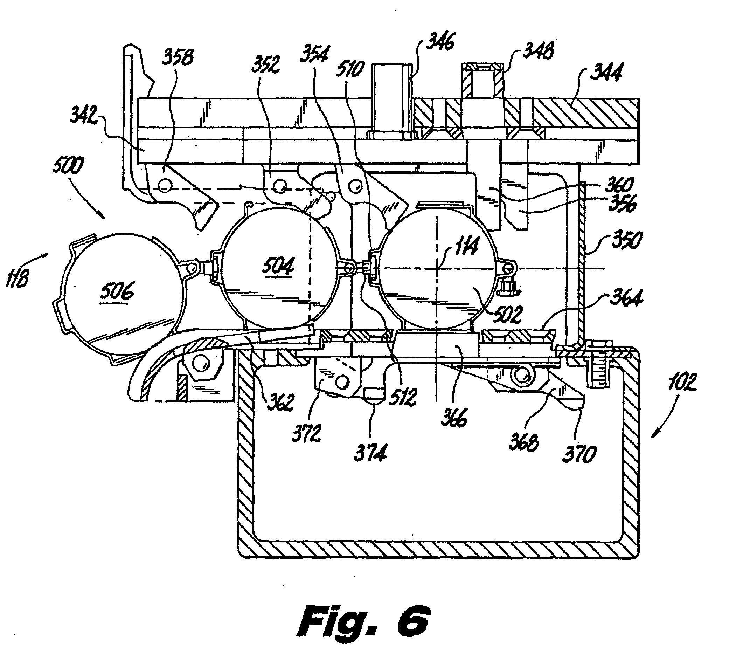

- Fig. 6: einen Schnitt wie in Fig. 4, bei Zündung der Patrone,

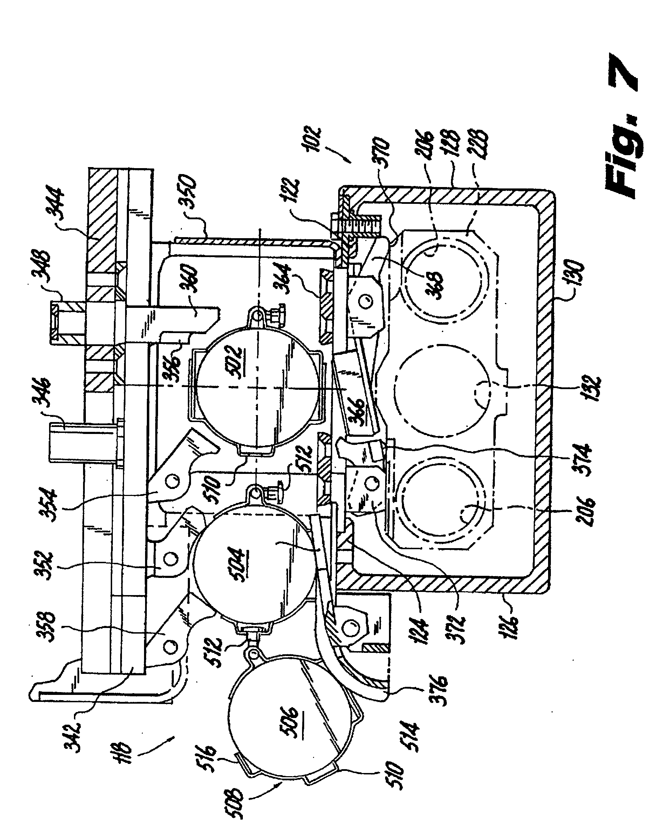

- Fig. 7: einen Schnitt wie in Fig. 4, bei Beginn des Rücklaufes des Masseverschlusses,

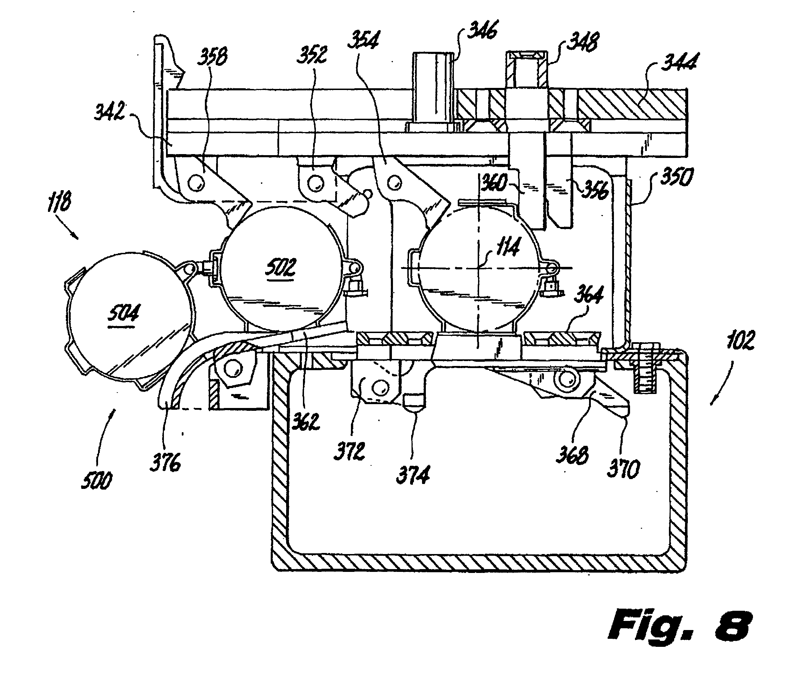

- Fig. 8: einen Schnitt wie in Fig. 4, beim Ausziehen der abgefeuerten Patronenhülse,

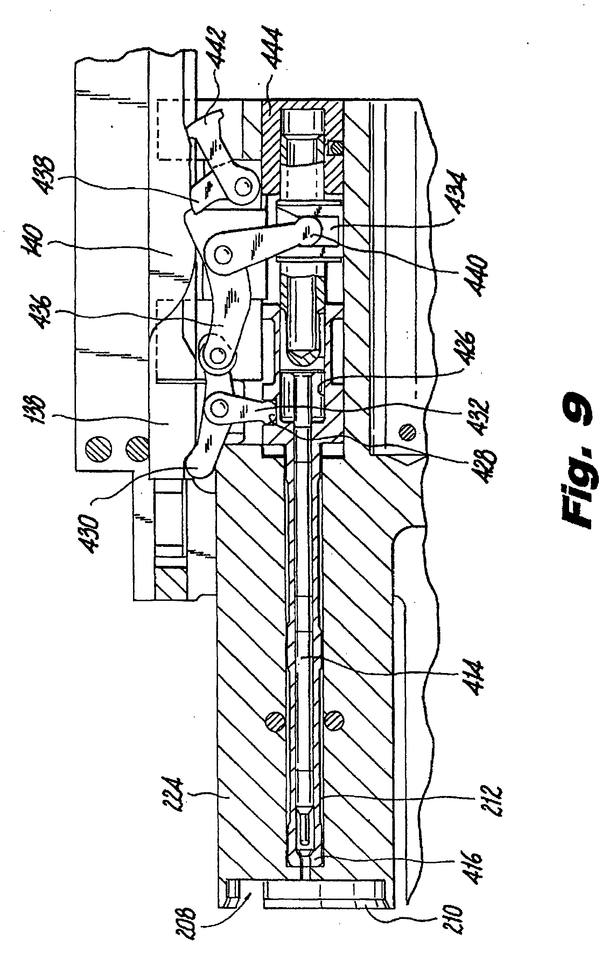

- Fig. 9: einen Teilschnitt durch den Verschlußkopf des Masseverschlusses, und

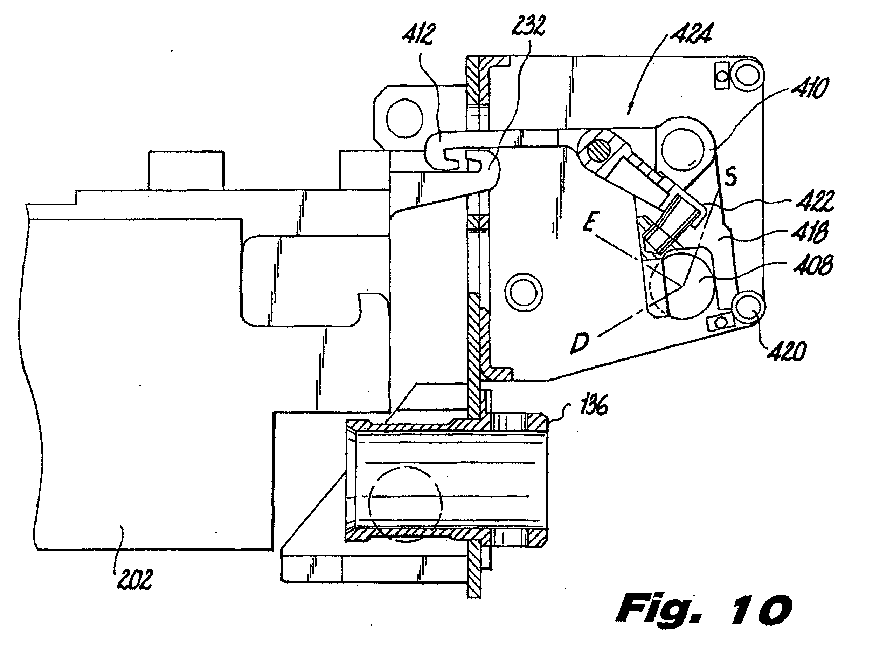

- Fig. 10: eine schematische, teilweise geschnittene Darstellung der Verschlußfangeinrichtung.

In den Figuren bedeuten gleiche Bezugszeichen durchgehend gleiche Bauteile oder Elemente.

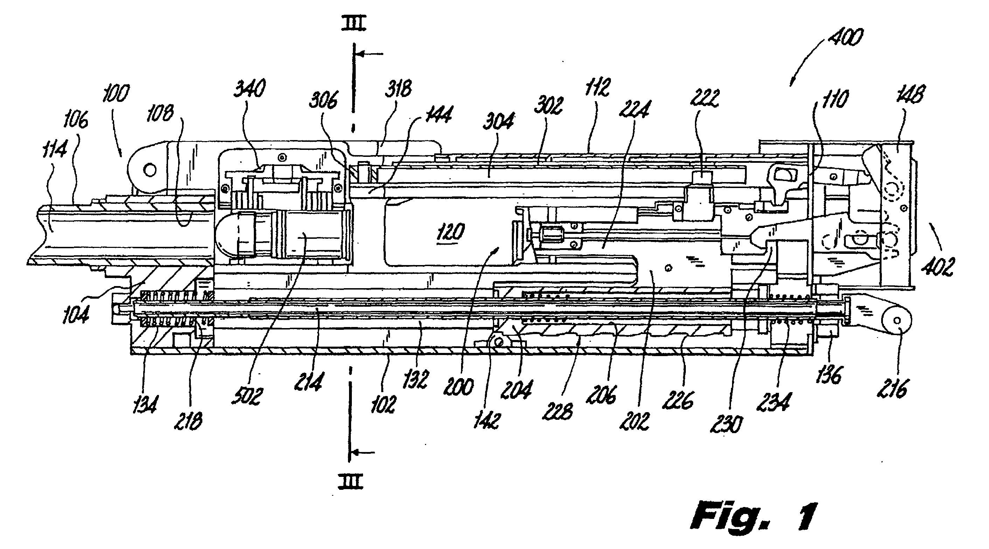

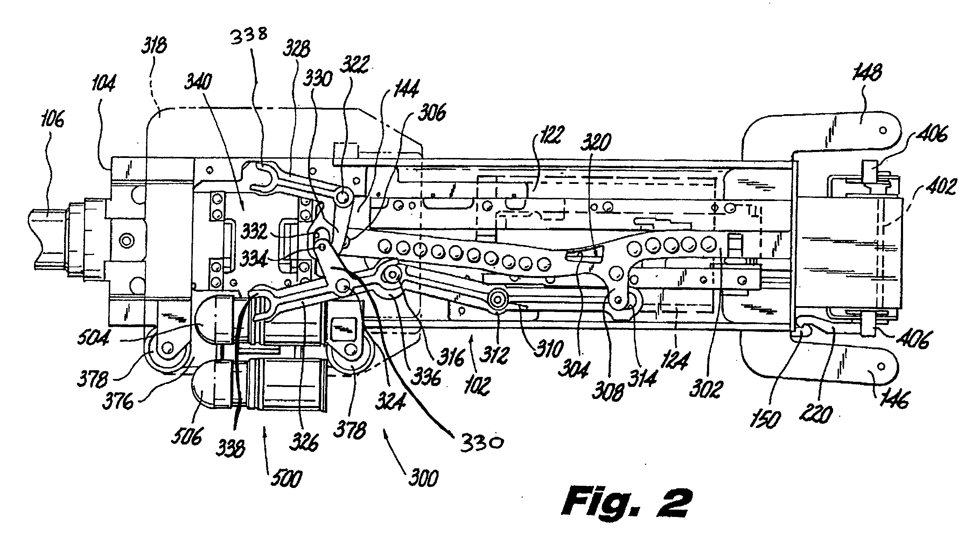

Der in den Übersichtsdarstellungen der Fig. 1 und 2 gezeigte Granatwerfer besteht im wesentlichen aus einer Gehäusegruppe 100, einer Verschlußgruppe mit Federung und Handhabungseinrichtung 200, einer Zubringergruppe mit Steuerung 300 und einer Abzugseinrichtungsgruppe 400. Der in den Granatwerfer eingeführte Patronengurt ist mit 500 bezeichnet, ist an sich bekannt und bildet als solcher keinen Bestandteil der Waffe.

### Der Patronengurt 500:

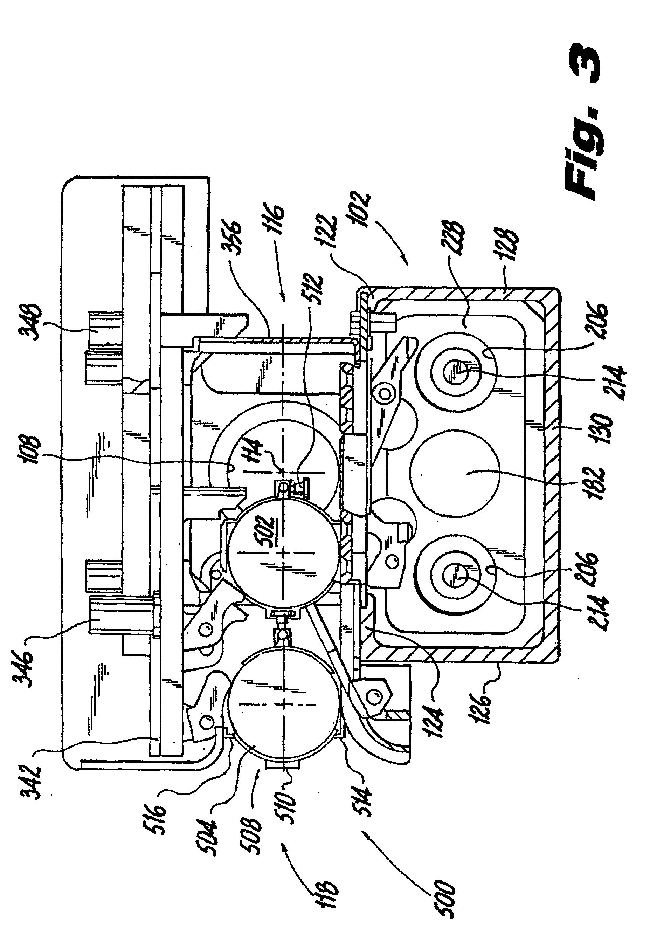

Zum vollen Verständnis der Waffe soll aber zunächst der bekannte Patronengurt in Erinnerung gerufen werden, wobei auf die Figuren 1 und 3 Bezug genommen wird. In den übrigen Figuren der Zeichnung sind die den Patronengurt betreffenden Bezugszeichen weggelassen, um nicht zu verwirren.

Der Patronengurt enthält eine vorderste Patrone 502, eine erste nachfolgende Patrone 504 und weitere Patronen 506 (nur eine in Fig. 2 gezeigt).

Die Patronen 502, 504 und 506 weisen jeweils ein Geschoß und eine Patronenhülse auf, die am hinteren Ende einen flanschartig überstehenden Rand aufweist sowie den Zündsatz und die Treibladung aufnimmt.

Jede Patronenhülse trägt ein sie wie eine Rohrschelle umschließendes, aus einen Blechband gebildetes Gurtglied 508. In Fig. 2 sind die Gurtglieder 508 weggelassen.

Das Gurtglied weist an der Ober- und Unterseite der Patrone 502, 504 jeweils einen breiten, abgeflachten Vorsprung 516 bzw. 514 auf, auf der einen (in Fig. 3 linken) Seite einen schmalen, abgeflachten Vorsprung 510, der ein Langloch mit erweitertem Ende aufweist, und auf der anderen Seite einen Vorsprung mit einem gelenkig an diesem angebrachten Anlenkzapfen 512, der ein verdicktes freies Ende aufweist.

Der Anlenkzapfen 512 sitzt bei dem zusammengesetzten Gurt im Langloch des Vorsprungs 510 des benachbarten Gurtgliedes 508 und hintergreift dieses mit seinem verdickten Ende.

Werden benachbarte Patronen gegeneinander verschoben, dann gelangt das verdickte Ende des Anlenkzapfens 512 vor das erweiterte Ende des ihn aufnehmenden Langloches, so daß die beiden benachbarten Patronen 502, 504 auseinanderbewegt werden können. So erfolgt das Entgurten; das Gurtglied 508 verbleibt auch an der entgurteten Patrone.

Das Gurtglied 508 sitzt bei der unabgeschossenen Patrone etwa an der vorderen Hälfte der Patronenhülse und umschließt diese stramm.

Wird die Patrone in ein Patronenlager 108 eingeführt, dann sitzt das Gurtglied auf dem hinteren Ende des Patronenlagers 108 auf und wird bis gegen den Patronenrand nach hinten geschoben. Die Patrone läßt sich somit nur soweit in das Patronenlager 108 einführen, daß der Patronenrand um einen Abstand vom hinteren Ende des Patronenlagers 108 getrennt ist, der der axialen Länge des Gurtgliedes 508 entspricht.

Die Patronenhülse ist so aufgebaut, daß sie dem Gasdruck beim Abfeuern standhält, obwohl sie nicht gänzlich in das Patronenlager 108 eingeführt ist.

Es folgt nun die Beschreibung des bevorzugten Ausführungsbeispiels des erfindungsgemäßen Granatwerfers:

### Die Gehäusegruppe 100:

Das Hauptteil der Gehäusegruppe ist von einer stranggepreßten Hohlprofilstange 102 gebildet, die im folgenden kurz "Gehäuse" genannt wird und die im wesentlichen einen Querschnitt mit zwei parallelen Seitenschenkeln aufweist, die einstückig an ihrem unteren Ende und etwa in ihrer Mitte durch jeweils einen geraden, rechtwinklig angesetzten Quersteg verbunden sind.

Das Gehäuse 102 weist somit eine linke Gehäusewand 126, eine rechte Gehäusewand 128 und einen Gehäuseboden 130 auf.

Die Längsmittellinie oder Längsmitte des Gehäuses ist mit 114 bezeichnet.

Das Gehäuse 102 wird durch Ablängen und nachfolgendes spanendes Bearbeiten einer stranggepreßten Hohlprofilstange gebildet, wobei infolge der spanenden Bearbeitung eine vordere, querverlaufende Einfräsung gebildet ist, welche zum Einführen des Patronengurtes 500 dient, mit einer rechten Einführöffnung 116 und einer linken Einführöffnung 118, ferner eine in der rechten Gehäusewand ausgearbeitete Auswurföffnung 120, durch welche abgeschossene Patronenhülsen, Exerzierpatronen oder Patronenversager aus dem Gehäuse ausgeworfen werden, und eine längsverlaufende Einfräsung im oberen Quersteg, so daß von diesem ein in Fig. 2 und 3 gezeigter, rechter Gehäusesteg 122 und linker Gehäusesteg 124 gebildet ist; an jeder der einander zugewandten Kanten der beiden Gehäusestege 122, 124 ist jeweils eine Stahlleiste mit einer Steuerkurve angebracht, und zwar die Steuerkurve 138 für die Schlagbolzenhülse 416 an der rechten Kante und die Steuerkurve 140 zum Steuern des Abschlagens des Schlagbolzens 414 an der linken Kante.

Dort, wo die von den beiden Gehäusestegen 122, 124 begrenzte Aussparung im oberen Quersteg nicht erforderlich ist, bleibt dieser stehen, etwa bei der Brücke 144.

Das Gehäuse 102 ist hartanodisiert, um eine geeignete Einfärbung (Tarnfarbe) und Oberflächengüte (Abriebbeständigkeit und Gleitverhalten) zu erhalten.

Im vorderen Ende des Gehäuses 102 ist ein Stahlblock 104 fest angebracht, der das auf die Längsmitte 114 zentrierte Rohr 106 mit dem Patronenlager 108 trägt.

Der Stahlblock 104 weist unterhalb und beiderseits des Rohres 106 jeweils eine nach hinten offene Sack-Aufnahmebohrung 134 auf, die jeweils von einem Federführungsstab 214 durchsetzt ist und eine auf diesem sitzende und abgestützte Pufferfeder 218 aufnimmt.

Die Pufferfeder reicht nach hinten bis in die vergrößerte Mündung der Aufnahmebohrung 134; diese Mündung ist so bemessen, daß sie jeweils einen Vorsprung 204 des Verschlußträgers 228 des Masseverschlusses 202 aufnehmen kann, wenn sich dieser bis ganz nach vorne bewegt (beim Abschlagen ohne Patrone).

In den Boden der Sackbohrung 134 mündet eine abgesetzte Abstütz- und Aufnahme-Durchgangsbohrung ein, in der der mit einer Führungsringwulst und einem Endzapfen ausgebildete Federführungsstab 214 im wesentlichen spielfrei aufgenommen ist. Hierbei ist das freie, vordere Ende des Endzapfens abgerundet, so daß der Federführungsstab, wenn er nach vorne in die Aufnahmebohrung 134 bewegt wird, sich selbst ausrichten kann.

In der Mitte zwischen den beiden Aufnahmebohrungen ist das Gehäuse 102 der Länge nach von einem Paß-Rundstab 132 (Fig. 1, angedeutet in Fig. 3) durchsetzt, der im Stahlblock 104 befestigt ist und der den Masseverschluß 202 bei seiner Bewegung führt.

An der Rückseite des Gehäuses 102 ist dieses durch eine Endabdeckung 110 verschlossen, in der zwei Führungen 136 für die Federführungsstäbe 214 sitzen und in der der Paß-Rundstab 132 abgestützt ist.

Die Endabdeckung trägt einen Teil der Abzugseinrichtungsgruppe 400 und ist zusammen mit dieser und der Verschlußgruppe 200 nach hinten abnehmbar.

Die Oberseite des Gehäuses 102 ist von einem abnehmbaren Gehäusedeckel 112 abgedeckt, der sich von der Endabdeckung 110 bis etwa zur Brücke 144 erstreckt.

Etwa in der Mitte der Länge des Gehäuses 102 ist an der Innenseite des Gehäusebodens 130 eine Ratschenklinke 142 um eine Querachse schwenkbar angebracht und durch eine Federung (nicht gezeigt) so belastet, daß sie danach trachtet, eine im wesentlichen aufrechte Lage einzunehmen.

Am Gehäuse 102 sind noch weitere, hier im einzelnen nicht dargestellte Teile befestigt, etwa ein Ausstoßer an der Innenseite der linken Gehäusewand 126, eine Montage für eine Visiereinrichtung an der Außenseite der linken Gehäusewand 126, jeweils eine Halterung für einen Patronenkasten außen an der linken oder rechten Gehäusewand 126, 128 im Bereich der linken und rechten Einführöffnung 118, 116, eine Halterung zum Anbringen einer Lafette außen an der linken und rechten Gehäusewand 126, 128 und/oder am Gehäuseboden 130 usw.

Außerdem ist am hinteren Ende außen an der linken und rechten Gehäusewand 126, 128 jeweils ein oberer und unterer, sich nach hinten und außen erstreckender Haltebügel angebracht; die Enden der übereinanderliegenden Haltebügel sind jeweils durch einen insgesamt vertikalen linken bzw. rechten Handgriff 146, 148 verbunden.

Das Ergreifen eines oder beider Handgriffe 146, 148 erlaubt das Richten und Feuern des Granatwerfers in üblicher Weise.

Schließlich ist an der Rückseite der linken Gehäusewand 126 unten und außen ein sich nach hinten erstreckender Fortsatz angebracht, der an seinem hinteren Ende einen einwärts weisenden Rastvorsprung 150 aufweist, aber insgesamt so angebracht ist, daß er das Entnehmen und Einsetzen der Verschlußgruppe 200 nicht behindert.

### Die Verschlußgruppe 200:

Die Verschlußgruppe 200 weist einen Masseverschluß 202 auf, der aus einem zur Längsmitte 114 koaxialen Verschlußkopf 224 und einem zu dieser parallelen Verschlußträger 228 gebildet ist, die übereinanderliegen und an ihrer Rückseite einstückig miteinander verbunden sind.

Der Verschlußkopf 224 weist an seiner Vorderseite einen Stoßboden 208 auf, der an der rechten Seite von einem üblichen, abgefederten, nach vorne überstehenden Auszieher 210 begrenzt ist.

Diesem gegenüberliegend ist auch an der linken Seite ein Auszieher (nicht gezeigt) angeordnet, um auch bei Erschütterungen der Waffe ein störungsfreies Ausziehen der Patronenhülse durch den vom Patronengurt 500 eingenommenen Bereich hindurch bis vor die Auswurföffnung 120 sicherzustellen; dieser linke Auszieher wird beim Verschlußrücklauf durch einen gehäusefesten Anschlag geöffnet und gibt den Rand der ausgezogenen Patronenhülse frei, kurz bevor dieser gegen den ebenfalls gehäusefesten Ausstoßer aufläuft.

Der Verschlußkopf 224 weist koaxial zur Längsmitte 114 eine Axialbohrung 212 auf (sh. Fig. 7), die als nach hinten offene Sackbohrung ausgebildet ist, deren Boden in üblicher Weise von einem Durchtrittskanal für die Spitze des Schlagbolzens 414 durchsetzt ist.

Diese Axialbohrung 212 nimmt die schon oben angesprochene Schlagbolzenhülse 416, den Schlagbolzen 414 und dessen Schlagfeder (nicht gezeigt) auf.

Der Verschlußträger 228 weist drei Bohrungen auf: eine (nicht gezeigte) Paßbohrung, die dazu eingerichtet ist, im wesentlichen spielfrei auf dem Paß-Rundstab 132 zu gleiten, und zwei nach hinten offene Schließfeder-Aufnahme-Sackbohrungen 206, die zu jeweils einer der Aufnahmebohrungen 134 koaxial sind.

Der Boden dieser Schließfeder-Aufnahme-Sackbohrungen 206 ist jeweils von einer kleineren Bohrung durchsetzt, durch die sich jeweils ein Federführungsstab 214 hindurch erstreckt.

Auf den hinteren Abschnitt eines jeden Federführungsstabes 214 ist jeweils eine Schließfeder 234 aufgeschoben, die als wendelförmige Druckfeder ausgebildet ist.

Jede dieser Schließfedern stützt sich vorne gegen den Boden der zugehörigen Schließfeder-Aufnahmebohrung 206 und hinten gegen die Federstabführung 136 ab.

An der Vorderseite des Verschlußträgers 228 sind die schon oben erwähnten Vorsprünge 204 ausgebildet.

Wie bei der Erläuterung der Gehäusegruppe 100 dargelegt, erstrecken sich die Federführungsstäbe 214 im schußbereiten Zustand des Granatwerfers nach vorne bis in die entsprechenden Ausbildungen einer zugehörigen Aufnahmebohrung 134 im Stahlblock 104, in der dann auch eine auf den Federführungsstab 214 aufgeschobene Pufferfeder 218 aufgenommen ist.

Diese Pufferfeder 218 kann sich entweder unmittelbar gegen den Boden der Aufnahmebohrung 134 oder gegen einen Radialvorsprung des Federführungsstabes 214 abstützen.

Beim Zurückziehen des Federführungsstabes 214 wird die Pufferfeder 218 entweder durch die vor dieser am Federführungsstab 214 ausgebildete Führungsringwulst oder durch ihre Abstützung am Federführungsstab 214 selbst mitgenommen.

Die beiden Federführungsstäbe 214 erstrecken sich durch die Federstabführungen 136 hindurch nach hinten und sind dort durch einen Spanngriff 216 fest miteinander verbunden, der sich unter den unteren Enden des rechten und linken Handgriffs 148, 146 quer bzw. horizontal erstreckt.

Um den Verschluß 202 zu spannen, wird der Spanngriff 216 hinlänglich weit horizontal nach hinten aus dem Gehäuse 102 herausgezogen und wieder bis zum Anschlag nach vorne zurückgeschoben. Dabei ergreift die eine Hand des Schützen den dieser entsprechenden Handgriff 146 oder 148 zur Abstützung, während die andere Hand den Spanngriff 216 betätigt. Somit ist ein Spannen der Waffe möglich, ohne daß sich der Schütze über die Waffe beugen muß und ohne daß auf die Waffe Kräfte aufgebracht werden, die geeignet sind, ihre gegebenenfalls vorher erfolgte Einjustierung auf ein Ziel zu beeinträchtigen.

Im Bereich des linken Endes des Spanngriffes 216 ist an diesem ein um eine vertikale Achse schwenkbarer, federnd nach außen gedrückter Auslösehebel 220 angebracht, der so angeordnet ist, daß er bei voll nach vorne geschobenem Spanngriff 216 den Rastvorsprung 150 des Gehäuses 102 sperrend hintergreift. Dabei sind die einander zugewandten Kanten von Rastvorsprung 150 und/oder Auslösehebel 220 so abgeschrägt, daß sie ineinander einrasten, wenn sie gegeneinander bewegt werden.

Der Auslösehebel ist mit einer (nicht gezeigten) Verlängerung versehen, die so am Spannhebel 216 angeordnet ist, daß sie bei dessen Ergreifen ohne weiteres mit ergriffen werden kann, so daß die vom Rastvorsprung 150 und dem Auslösehebel 220 gebildete lösbare Sperre gelöst wird und den Spannvorgang nicht behindert.

Wird der Spanngriff 216 dagegen bis ganz nach vorne geschoben und losgelassen, dann tritt diese lösbare Sperre 150, 220 in Eingriff und verhindert jegliches unerwünschte Freikommen des Spannngriffs 216.

Der Verschlußkopf 224 trägt ferner hinten an seiner Oberseite einen mittig angeordneten Kurvenhebel-Mitnehmer 222, der bevorzugt als eine um eine vertikale Achse drehbare, gehärtete Rolle ausgebildet ist.

An der Rückseite des Masseverschlusses 202 ist ferner ein Abzugsstollen 230 angeordnet, der als querverlaufende, sich nach oben erstreckende Leiste ausgebildet ist, deren Oberkante knapp unter der Längsmitte 114 liegt und deren Vorderseite eine im wesentlichen vertikal abfallende Querfläche bildet.

Der Abzugsstollen 230 ist so ausgebildet, daß er von einer Nase am vorderen Ende eines in der Abzugseinrichtung 402 um eine horizontale Achse schwenkbar gelagerten Abzugshebels 404 von oben her umgriffen wird. Wird der Abzugshebel 404 mit seiner Nase nach oben weggeschwenkt, dann gibt er den Abzugsstollen 230 und damit den Masseverschluß 202 frei, so daß dieser unter der Wirkung der Schließfedern 234 nach vorne schnellen kann.

Oberhalb des Abzugsstollens 230 ist ein hakenartiger, nach vorne offener und von oben her ergreifbarer Fangvorsprung 232 ausgebildet, der in Fig. 10 gezeigt ist und in Zusammenhang mit der Abzugseinrichtungsgruppe 400 weiter unten erläutert werden wird.

An der Unterseite des Verschlußträgers 228 ist eine Reihe in Längsrichtung hintereinanderliegender Ratschenzähne 226 angeordnet, deren vordere Zahnflanken sich vertikal erstrecken, deren Zahnspitzen horizontal abgeflacht sind und deren hintere Zahnflanken gegenüber der Horizontalen jeweils nur um einen sehr flachen Winkel, etwa 10°, geneigt sind.

Der Grund zwischen der hinteren Zahnflanke eines vorderen Ratschenzahnes 226 und der vorderen Zahnflanke eines nachfolgenden Ratschenzahnes 226 ist horizontal abgeflacht.

Der vertikale Abstand zwischen den Ratschenzähnen 226 und der am Gehäuse 102 schwenkbar angebrachten Ratschenklinke 142 ist so bemessen, daß die Ratschenklinke 142 sich unter den Ratschenzähnen 226 nur bis zu einer solchen Schräglage aufrichten kann, daß sie imstande ist, dann, wenn sie nach hinten gekippt ist, sperrend gegen eine der vorderen Zahnflanken anzuliegen, dagegen dann, wenn sie nach vorne gekippt ist, die Ratschenzähne 226 unbehindert über sich weggleiten zu lassen.

Die Länge der zahnstangenartigen Reihe von Ratschenzähnen 226 und damit des Verschlußträgers 228 ist so bemessen, daß diese Reihe voll über die Ratschenklinke 142 nach vorne oder hinten hinweggelaufen ist, wenn sich der Masseverschluß 202 in seiner vordersten oder hintersten Lage befindet.

In jeder dieser Lagen ist es der Ratschenklinke 142 somit gestattet, sich durch Federwirkung vollständig aufzurichten, so daß sie beim Rücklauf des Masseverschlusses 202 nach hinten, beim Vorlauf dagegen nach vorne gekippt wird.

In der in Fig. 1 gezeigten Stellung befindet sich der Masseverschluß 202 in seiner Auslöselage, in der er durch den Eingriff des Abzugshebels 404 in den Abzugsstollen 230 in seiner Lage festgehalten wird. Diese Auslöselage befindet sich ein wenig vor dem hintersten Ende des Rücklaufes, wo es der Ratschenklinke gestattet ist, sich ganz aufzurichten. Nun, in der Auslöselage, liegt das vordere Ende der zahnstangenartigen Reihe von Ratschenzähnen 226 von hinten her gegen die Ratschenklinke 142 an und kippt sie nach vorne.

Wird nun der Masseverschluß 202 freigegeben, dann läuft er unbehindert über die Ratschenklinke 142 hinweg, bis er in seine vorderste Lage gelangt. Hier richtet sich die Ratschenklinke 142 hinter der zahnstangenartigen Reihe wieder auf und wird beim Rücklauf nach hinten gekippt.

Wird nun aus irgendeinem Grund der Rücklauf unterbrochen, etwa weil eine Patrone mit ungenügendem Rückstoß abgefeuert wurde oder der Schütze beim Spannen des Masseverschlusses 202 behindert wurde, so daß dessen Rückwärtsbewegung schon vor der Auslöselage abgebrochen wird, dann kann der Masseverschluß 202 nicht nach vorne schnellen. Dies ist erst dann möglich, wenn die Rücklaufbewegung mittels des Spanngriffs 216 vervollständigt wurde.

Somit wird ein unerwünschtes Abfeuern verhindert, das möglicherweise, etwa beim Loslassen des Spanngriffes 216, stattfinden könnte, weil in der dann erreichten Stellung des Masseverschlusses 202 (vor der Auslöselage) der Abzugshebel 404 noch nicht in den Abzugsstollen 230 eingreifen und den Masseverschluß 202 festhalten kann.

Das Aufhalten des Masseverschlusses 202 in einer Lage vor der Auslöselage ist bei vielen Waffen, etwa den meisten Maschinenpistolen oder Maschinengewehren, zweckmäßig, ist aber bei der dargestellten Waffe darüber hinaus deshalb von grundlegender Bedeutung, weil bei dieser Waffe die Zündung der Patrone 502 nicht erst dann erfolgt, wenn sie voll in das Patronenlager 108 eingeführt wurde, sondern schon einen kurzen, genau festgelegten Zeitraum vorher, wenn sich Patrone 502 und Masseverschluß 202 in voller Bewegung befinden, wobei in an sich bekannter Weise die dann aufgebrachte kinetische Energie zum Abfangen eines Teiles des Rückstoßes dient, der durch den Abschuß der Patrone 502 entsteht.

Da aber, wie eingangs erwähnt, die Patrone 502 nicht voll ins Patronenlager 108 eingeführt werden kann, sondern um einen beträchtlichen Abstand (axiale Länge des Gurtgliedes 508) aus dem Patronenlager 108 herausragt, wenn sie gezündet wird, werden die genaue Lage des Masseverschlusses 202 und dessen eng tolerierte Geschwindigkeit jeweils zum Zündzeitpunkt zu höchst kritischen Werten. Das beschriebene Ratschengesperre 142, 226 sorgt dafür, daß die Geschwindigkeit des Masseverschlusses 202 sich beim Zünden der Patrone 502 zuverlässig innerhalb der zulässigen Toleranz befindet.

### Die Zubringergruppe 300:

Die Zubringergruppe 300 besteht aus der eigentlichen Zubringereinrichtung, ihrer Steuerung und dem Gurteinlauf; die Steuerung ihrerseits besteht aus den gehäuseseitigen Steuerelementen und den in einem Zubringerdeckel 318 angeordneten Steuerelementen.

Die gehäuseseitigen Steuerelemente bestehen aus einem Kurvenhebel 302 und einem zweiarmigen Umlenkhebel 310, die beide jeweils um eine vertikale Achse schwenkbar im Gehäuse 102 gelagert sind.

Der Kurvenhebel 302 ist aus einem nach unten offenen U-Profilstab gebildet, dessen nach oben gewandter Boden zur Gewichtserleichterung und zur Bildung von Schmutz-Aufnahmeräumen gelocht ist. Der U-Profilstab ist insgesamt, von oben betrachtet, schwach S-förmig gebogen. Seine nach unten gerichtete Höhlung bildet eine in einer horizontalen Ebene liegende, gekrümmte Steuerkurve 304, in welcher der Kurvenhebel-Mitnehmer 222, der mittig, oben und hinten auf dem Verschlußkopf 224 sitzt, nahezu spielfrei gleiten kann.

Der Kurvenhebel 302 ist an seiner Vorderseite (Oberseite seiner S-Form) an einem Lagerzapfen 306 schwenkbar gelagert, der fest, mittig und aufrecht in der Brücke 144 angebracht ist und von dieser nach oben absteht.

Bei der geradlinigen Vor- und Rückwärtsbesegung des Masseverschlusses 202 und damit des Kurvenhebel-Mitnehmers 222 läuft dieser in der Steuerkurve 304 entlang und veranlaßt somit den Kurvenhebel 302 zu einer Schwenkbewegung, deren Ablauf von der Krümmung der Steuerkurve 304 gesteuert wird.

Der Kurvenhebel 302 weist kurz hinter der Mitte seiner Längenerstreckung eine nach rechts (zum rechten Gehäusesteg 122) hin geöffnete Kurvenhebelaussparung 320 auf, die sich in den Boden und die rechte Seitenwand des Kurvenhebels erstreckt, aber die Wirkung der Steuerkurve 304 in keiner Weise beeinträchtigt.

In diese Kurvenhebelaussparung 320 greift ein Arretierhebel (nicht gezeigt) ein, der mit einem abgefederten Tastfinger (nicht gezeigt) gekoppelt ist, der vom geschlossenen Zubringerdeckel 318 niedergehalten wird.

Normalerweise befindet sich dieser Arretierhebel außer Eingriff mit der Kurvenhebelaussparung 320 und übt somit keinerlei Wirkung aus.

Wird aber der Zubringerdeckel 318 geöffnet, etwa zum Einlegen eines Patronengurtes 500 oder zum Beseitigen einer Ladehemmung, dann kann der Tastfinger federnd ausfahren und nimmt den Arretierhebel mit, der dann in die Kurvenhebelaussparung 320 eingreift und sich an deren Rand abstützt.

Läßt nun der Schütze versehentlich den Masseverschluß 202 abschlagen, dann wird dieser'durch das Auflaufen des Kurvenhebel-Mitnehmers 222 gegen den Arretierhebel aufgefangen, so daß der Masseverschluß 202 nicht die Hand des Richt- oder Ladeschützen, die sich gerade im Bereich unmittelbar hinter dem Patronenlager 108 befinden kann, erreichen und verletzen kann. Die Erschütterung bei diesem Auflaufen ist so stark, daß sie vom Schützen bemerkt wird, der dann einfach nur den Spanngriff 216 zurückzuziehen braucht. Wegen der stark abgeschrägten Hinterkanten der Ratschenzähne 226 kann der Masseverschluß 202 nach hinten bewegt werden, obwohl die Ratschenklinke 142 nach vorne gekippt ist.

Der Kurvenhebel 302 weist unmittelbar hinter der Kurvenhebelaussparung 320 einen etwa rechtwinklig nach links abstehenden Seitenschenkel 308 auf, dessen freies Ende einen abwärtsgerichteten Zapfen trägt, der passend in ein Langloch 312 im hinteren Ende des zweiarmigen Umlenkhebels 310 eingreift.

Dieser Umlenkhebel 310 ist auf der Höhe des Kurvenhebels 302 zwischen diesem und der linken Gehäusewand 126 angeordnet und erstreckt sich etwa in Längsrichtung des Gehäuses 102.

Der zweiarmige Umlenkhebel 310 ist aus zwei gleichlangen Armen gebildet, die zwischeneinander einen sehr stumpfen Winkel von etwa 165° einschließen.

In seiner Mitte ist der zweiarmige Umlenkhebel 310 schwenkbar an einem aufrechten Lagerzapfen 312 angebracht, der fest und aufrecht nach oben abstehend am linken Gehäusesteg 124 befestigt ist.

Am vorderen, freien Ende des zweiarmigen Umlenkhebels 310 ist ein Umlenkhebelzapfen 316 angebracht, der von der Oberseite des Umlenkhebels 310 nach oben absteht. Dieser Umlenkhebelzapfen 316 befindet sich ein wenig hinter der Brücke 144.

Der Gehäusedeckel 112 deckt alle gehäuseseitigen Steuerelemente (Kurvenhebel 312, Umlenkhebel 310) von oben her staubdicht ab; lediglich das vorderste Ende des Umlenkhebels 310 zusammen mit dem Umlenkhebelzapfen 316 steht nach vorne über die Vorderkante des Gehäusedeckels 112 vor.

Vor dem Gehäusedeckel 112 ist ein Zubringerdeckel 318 auf dem Gehäuse 102 angebracht und mittels einer Scharnieranordnung, die auf der Oberseite des Stahlblocks 104 ausgebildet ist, um eine horizontale Querachse schwenkbar befestigt. Der Zubringerdeckel 318 ist in Fig. 1 gezeigt, in Fig. 2 nur in seinem Umriß strichpunktiert angedeutet und in Fig. 3 schematisiert. In allen drei Figuren befindet sich der Zubringerdeckel 318 in seinem geschlossenen Zustand, in dem er durch eine lösbare Sperre gehalten wird.

Der Zubringerdeckel 318 ist um fast einen Patronendurchmesser breiter als das Gehäuse, erstreckt sich nach hinten bis über die Vorderkante des Gehäusedeckels 112 hinaus und schirmt somit wie ein Vordach die jeweilige Einführöffnung 116, 118 im Gehäuse 102 gegenüber herabfallendem Schmutz (Schlamm, Sand, Erde) ab.

Ferner verdeckt der Zubringerdeckel 318 den Spalt zwischen der Brücke 144 und der Vorderkante des Gehäusedeckels 112.

Der Zubringerdeckel 318 ist als ein nach unten offener, flacher Behälter ausgebildet. In dem Teil des Zubringerdeckels 318, der in dessen Schließstellung über der Brücke 144 liegt, sind zwei vertikale Lagerzapfen 322, 324 fest angebracht, deren Achsen mit jeweils gleichem Abstand von der Längsmitte 114 in einer gemeinsamen, zu dieser senkrechten Ebene liegen.

Auf dem linken Lagerzapfen 324 ist ein im wesentlichen gerader, erster Steuerhebel 326 schwenkbar gelagert, der in sich der in Fig. 2 gezeigten Lage des Masseverschlusses 202 (Auslöselage) um einen Winkel von etwa 15° vom Lagerzapfen 324 aus nach vorne und auswärts erstreckt.

Der erste Steuerhebel 326 ist nach hinten verlängert und endet in einem hinteren Aufnahmemaul 336, das in lösbarem und kraftübertragendem Eingriff mit dem Umlenkhebelzapfen 316 steht.

Auch am vorderen Ende weist dieser erste Steuerhebel 326 ein Aufnahmemaul 338 auf, das in lösbarem kraftübertragendem Eingriff mit einem ersten Schieberzapfen 346 steht.

Der erste Steuerhebel 326 weist auch noch einen im wesentlichen horizontal und rechtwinklig vom Bereich des linken Lagerzapfens 324 abstehenden Steuerhebelschenkel 330 auf, der bis etwa über die Längsmitte 114 reicht und an seinem freien Ende einen sich vertikal nach unten erstreckenden Eingriffszapfen 334 trägt.

Auf dem rechten Lagerzapfen 322 ist ein im wesentlichen gerader, zweiter Steuerhebel 328 schwenkbar gelagert, der in sich der in Fig. 2 gezeigten Lage des Masseverschlusses 202 (Auslöselage) um einen Winkel von etwa 15° vom Lagerzapfen 324 aus nach vorne und auswärts erstreckt, und zwar symmetrisch zum ersten Steuerhebel 326.

Am vorderen Ende weist dieser zweite Steuerhebel 328 ein Aufnahmemaul 358 auf, das in lösbarem kraftübertragendem Eingriff mit einem zweiten Schieberzapfen 348 steht.

Der zweite Steuerhebel 328 ist etwa rechtwinklig abgewinkelt, wobei der Scheitel des Winkels im Bereich des rechten Lagerzapfens 322 liegt.

Der abgewinkelte Teil des zweiten Steuerhebels 328 bildet einen Steuerhebelschenkel 330, der bis etwa über die Längsmitte 114 reicht und an seinem freien Ende ein Langloch 332 aufweist, das den Eingriffszapfen 334 mit Gleitpassung aufnimmt und sich im wesentlichen quer zu dessen Bewegungsbahn beim Verschwenken des ersten Steuerhebels 326 erstreckt.

Der Eingriffszapfen 334 und das Langloch 332 bilden somit eine im wesentlichen spielfreie Zwangskoppelung, die dafür sorgt, daß der zweite Steuerhebel 328 genau gegenläufig der Bewegung des ersten Steuerhebels 326 bei dessen Schwenkbewegung folgt: schwenkt beispielsweise der hintere Teil des Kurvenhebels 302 in der Draufsicht der Fig. 2, in Schußrichtung gesehen, nach rechts, dann bewegen sich die beiden vorderen Aufnahmemäuler 338 der beiden Steuerhebel 326, 328 aufeinander mit gleicher Geschwindigkeit zu.

Die beiden Schieberzapfen 346, 348 (Fig. 3) sind bevorzugt als drehbar gelagerte Rollen ausgebildet, um die Reibung beim Eingriff in die Aufnahmemäuler 338 zu mindern.

Der Zubringerdeckel 318 nimmt aber nicht nur einen Teil der Steuerung, wie beschrieben, sondern auch den wesentlichen Teil der eigentlichen Zubringereinrichtung auf.

Diese weist einen ersten Schieber 342 und einen zweiten Schieber 344 (Fig. 4) auf, die beide horizontal und quer zur Längsmitte 114 verschieblich in einer Schieberführung 340 aufgenommen sind, die im Zubringerdeckel 318 enthalten ist.

Der erste Schieber 342 trägt nach oben abstehend den ersten Schieberzapfen 346, der zweite Schieber 344 (Fig. 4) ebenso den zweiten Schieberzapfen 348. Die beiden Schieberzapfen und deren Bewegungsbahnen liegen beide auf einer gemeinsamen, zur Längsmitte 114 senkrechten Ebene.

Diese Schieberführung 340 ist in ihrem Querschnitt quer zur Richtung der Schieberbewegungen so ausgebildet und ist soweit zerlegbar, daß die beiden Schieber 342, 344 herausgenommen und entgegen ihrer ursprünglichen Bewegungsrichtung wieder eingesetzt werden können.

Infolge des symmetrisch erfolgenden Antriebs durch die beiden Steuerhebel 326, 328 funktionieren die beiden Schieber 342, 344 auch in umgekehrter Ausrichtung, fördern dann aber den Patronengurt 500 in Gegenrichtung in die Waffe, also nicht durch die linke Einführöffnung 118, wie in Fig. 2 gezeigt, sondern durch die rechte Einführöffnung 116.

Die jeweils nicht benutzte Einführöffnung 116, 118 wird, wie aus Fig. 3 ersichtlich, von einer Blechplatte 350 oder sonstigen Abdeckung verschlossen, um das Eindringen von Schmutz in die Waffe zu verhindern.

Die weiteren Elemente der Zubringereinrichtung werden unter Bezugnahme auf die Fig. 4 bis 8 beschrieben und sind der Deutlichkeit halber nur in diesen Figuren mit Bezugszeichen versehen.

Der erste Schieber 342 weist, nach unten abstehend, am rechten Ende einen festen Anschlag 356 auf, am linken Ende eine Außenklinke 352 und etwa in der Mitte eine Innenklinke 354.

Der zweite Schieber 344 weist, nach unten abstehend, am rechten Ende eine feste Stütze 360 und am linken Ende eine Schwenkklinke 358 auf.

Die Klinken 352, 354 und 358 sind jeweils als nach unten vorstehende Finger ausgebildet, die an ihrem oberen Ende jeweils gegen die Kraft einer Federung nach oben und zu der durch die Blechplatte 350 verschlossenen Einführöffnung 116 hin jeweils um eine Achse schwenkbar sind, die parallel ist zur Längsmitte 114.

Durch die andere Einführöffnung 118 läuft der Patronengurt 500 in die Waffe hinein.

Die Unterkanten der Klinken 352, 354 und 358 sind so ausgebildet, daß sie, wenn sie nach unten vorstehen und in Einlaufrichtung des Patronengurtes 500 bewegt werden, dessen jeweils vorderste und gegebenenfalls zweite Patrone 502 und 504 hintergreifen und fördern.

Werden die Klinken 352, 354 und 358 jedoch entgegen der Einlaufrichtung bis an eine Patrone 504 heranbewegt, dann werden sie durch die jeweils auflaufende Patrone weggeschwenkt, so daß diese unter ihnen passieren kann.

Lediglich die Außenklinke 352 gelangt dann, wenn sich die beiden Schieber 342, 344 zwischen den beiden Relativlagen der Figuren 3 und 4 befinden, in Sperreingriff mit dem zweiten Schieber 344, so daß sie dann nicht wegschwenken kann, sondern entgegen der Einlaufrichtung des Patronengurtes 500 gegen die zweite Patrone 504 anläuft und sie (und damit den ganzen Patronengurt 500) ein wenig zurückschiebt, ohne wegzuschwenken.

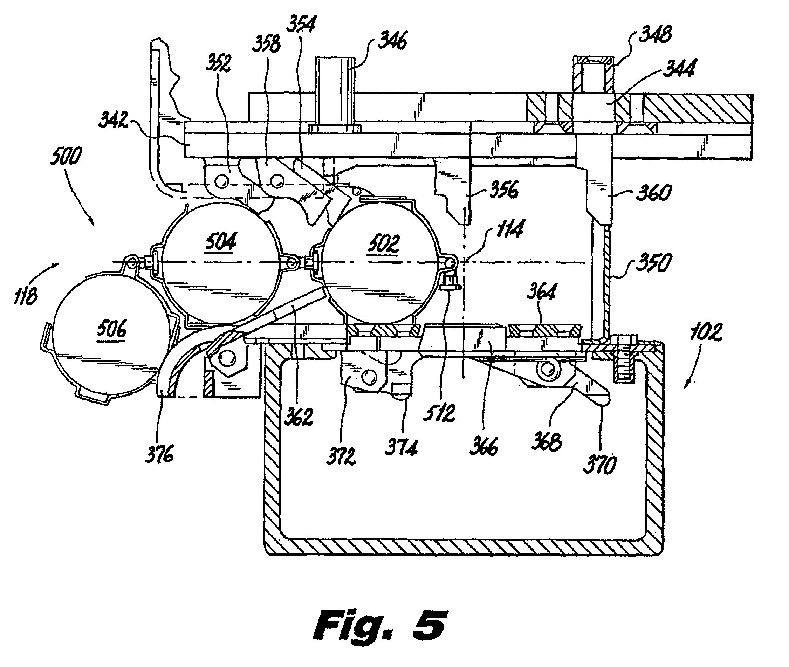

Am Gehäuse 102 ist unter der benutzten Einführöffnung 118 ein in diese hinein weisender Sperrhebel 362 mit seinem außenliegenden Ende um eine zur Längsmitte 114 parallele Achse schwenkbar gelagert, wird durch eine Federung bis in die in Fig. 4 und 5 gezeigte Lage angehoben und kann durch die über ihm weglaufenden Patrone 504 in die in Fig. 6 bis 8 gezeigte Lage niedergedrückt werden.

Die Wirkungsweise der von den beiden Schiebern 344, 346 getragenen Elemente und des Sperrhebels 362 wird nachfolgend kurz anhand der in den Figuren 4 bis 8 gezeigten Bewegungsfolge erläutert:

Fig. 4 zeigt die Lage des Patronengurtes 500 und der beiden Schieber 344, 346, wenn die Waffe nach Abgabe eines Schusses gespannt und schußbereit ist, sich der Masseverschluß 202 demnach in seiner Auslöselage befindet.

Der Sperrhebel 362 ist aufgestellt und stützt die erste Patrone 502 von außen her ab, die Schwenkklinke 358 ist gerade dabei, über diese Patrone 502 hinwegzulaufen und hintergreift sie bereits, hat aber noch nicht ihre voll aufrechte Lage erreicht. Die Außenklinke 352 wird gerade von der zweiten Patrone 504 nach oben weggeschwenkt, und die Innenklinke 354 befindet sich in voll aufrechter Lage.

Soll der Patronengurt 500 dagegen erst eingelegt werden, dann wird der Zubringerdeckel 318 aufgeklappt, wobei sich dann alle Klinken 352, 354 und.358 in voll aufrechter lage befinden, der Patronengurt 500 wird mit seiner vordersten Patrone 502 hinter das aufstehende, freie, zur Längsmitte 114 hin weisende Ende des Sperrhebels 362 gelegt und gegen dieses durch leichtes Anziehen am Patronengurt 500 angehalten und der Zubringerdeckel 318 wird wieder geschlossen.

Dann ist die Lage aller Elemente dieselbe wie in Fig. 4, mit der Ausnahme, daß sich die Schwenkklinke 358 in voll aufrechter Lage befindet und die erste Patrone 502 hintergreift.

Beginnt nun der Masseverschluß 202 mit seiner Vorwärtsbewegung, dann begingen die beiden Schieber 342, 344 eine solche Bewegung, daß sich die beiden Schieberzapfen 346, 348 aufeinander zu bewegen, bis sie die in Fig. 5 gezeigte Lage erreichen.

Die Innenklinke 354 hat sich mittlerweile in Einlaufrichtung des Patronengurtes 500 gegen die erste Patrone 502 heranbewegt, die Schwenkklinke 358 entgegen der Einlaufrichtung wegbewegt.

Bei der weiteren Bewegung schiebt die Innenklinke 354 die erste Patrone 502 bis vor das Patronenlager 108 (siehe Fig. 3), während die Schwenkklinke 358 nach außen über die zweite Patrone 504 hinwegläuft. Die feste Stütze 360 ist bis an die erste Patrone herangelaufen, die zwischen dieser festen Stütze 360, der Innenklinke 354 und Patronenauflagefingern 366, die weiter unten erläutert werden, in einer genau definierten Lage festgehalten wird. Die voll aufgerichtete Aussenklinke 352 liegt gegen die Seite der zweiten Patrone 504 an, die der ersten Patrone 502 zugewandt ist.

Der Abstand der beiden Schieberzapfen 346, 348 hat sein Minimum erreicht.

Nun erreicht der Verschlußkopf 224 mit dem Stoßboden 208 den Patronenboden und schiebt die erste Patrone 502 nach vorne.

Dabei verschiebt sich der Anlenkzapfen 512 am Gurtglied 508 der zweiten Patrone 504 im Langloch des Vorsprungs 510 am Gurtglied 508 der ersten Patrone. Die beiden Schieber 342, 344 kehren ihre Bewegungsrichtung um und beginnen, sich mit ihren Schieberzapfen 346, 348 auseinanderzubewegen.

In dieser Relativlage der beiden Schieber 342, 344 übergreift, wie bereits oben erörtert, der zweite Schieber 344 die Außenklinke 352 und hindert sie daran, zu schwenken.

Die Außenklinke 352 schiebt somit die zweite Patrone 504 entgegen der Einlaufrichtung von der ersten Patrone 502 weg, wobei der Anlenkzapfen 512 des Gurtglieds 508 der zweiten Patrone 504 aus der Erweiterung im Langloch des zugewandten Vorsprungs 510 herausgezogen wird.

Die zweite Patrone 504 bewegt sich weiter nach außen, bis sie an der Schwenkklinke 358 zum Stillstand gelangt (Lage der Fig. 7). Hierbei hat die zweite Patrone dem vorbeilaufenden Verschlüßkopf 224 Platz gemacht, ebenso wie die Innenklinke 354 und die feste Stütze 360, die beide von der ersten Patrone 502 zurückgefahren sind, um den Verschlußkopf 224 vorbeizulassen. Die seitliche Abstützung der Patrone 502 ist nun nicht mehr nötig, da sie sich mit dem vorderen Teil bereits im Patronenlager 108 befindet und mit dem Patronenboden am Stoßboden 208 gehalten ist.

Wenn die Patrone 502 abgefeuert wird, dann befinden sich alle Elemente der Zubringereinrichtung in der in Fig. 7 gezeigten Lage.

Nun beginnt der Verschlußrücklauf, und die bisher beschriebene Bewegungsfolge läuft in umgekehrter Reihenfolge wieder ab.

Beim Ausziehen der abgeschossenen Patronenhülse nähern sich die Innenklinke 354 und feste Stütze 360 an diese an und führen sie,

Dann bewegen sich die beiden Schieber 342, 344 mit ihren Schieberzapfen 346, 348 rasch auseinander, wobei die Schwenkklinke 358 die bisher zweite Patrone 504 und jetzt erste Patrone 502 bis in die Lage der Fig. 4 nachführt, wo sie vom Sperrhebel 362 hintergriffen wird.

Dabei verhindert der feste Anschlag 356 oder 360, daß die Patrone 502 zu weit gefördert wird.

An der Außenseite des Gehäuses 102 ist unter der linken Einlauföffnung 118 eine nach außen und unten gekrümmte Gurtführungsbühne 376 angebracht. Wird die rechte Einlauföffnung 116 für den Gurteinlauf benutzt, dann kann sie aufgrund ihres symmetrischen Aufbaus auch abgenommen, umgedreht und unter der rechten Einlauföffnung 116 angebracht werden.

Auch der Sperrhebel 362 kann vor der rechten Einlauföffnung 116 angebracht werden.

An die Gurtführungsbühne 376 schließt im Gehäuse 102 höhengleich ein horizontaler Führungstisch 364 an, der hinter dem Patronenlager 108 einen Durchbruch aufweist, der von einem höhengleichen Patronenauflagefinger 366 überbrückt ist.

Dieser ist rechts unter der angrenzenden Kante des Führungstisches 364 an einer zur Längsmitte 114 parallelen Achse schwenkbar gelagert und wird durch eine Feder nach oben gedrückt, wobei der genannte Durchbruch im Führungstisch 364 seine obere Endlage festlegt.

Der Patronenauflagefinger 366 ist nach rechts unten über die Lagerung hinaus durch einen Führungshebel 368 verlängert, dessen Ende einen Führungshebel-Mitnehmer 370 bildet.

Dieser Führungshebel-Mitnehmer 370 ist in Zuordnung zur Bewegung des Masseverschlusses 202 so angeordnet, daß dann, wenn die vorderste Patrone 502 in das Patronenlager 108 eingeführt werden soll, der Verschlußträger 228 (in Fig. 7 gestrichelt gezeigt) gegen den Führungshebel-Mitnehmer 370 anläuft und dabei den Patronenauflagefinger 366 soweit nach unten wegschwenkt (Fig. 7), daß sich die Patrone 502 trotz ihres überstehenden Randes und trotz des unteren, abgeflachten Vorsprungs 514 des Gurtglieds 508 genau koaxial zum Patronenlager 108 und damit zur Längsmitte ausrichten kann.

Um zu verhindern, daß der Patronenauflagefinger 366 unter der Einwirkung von Stößen unkontrolliert nach unten schwingt, ist unter dem linken Rand des genannten Durchbruchs im Führungstisch 364 ein Klemmhebel 372 um eine zur Längsmitte 114 parallele Achse schwenkbar angebracht, der das freie Ende des Patronenauflagefingers 366 untergreift und damit festlegt.

Der Klemmhebel 372 ist an seiner Unterseite mit einem Klemmhebel-Mitnehmer 374 versehen, der vom Verschlußträger 228 ebenso wie der Führungshebel-Mitnehmer 370 hochgedrückt werden kann, um den Führungshebel 368 freizugeben (Fig. 7).

Wie ersichtlich, ist der Patronenauflagefinger 366 nur dann weggeschwenkt, wenn die Patrone 502 gerade in das Patronenlager 108 eingeführt oder deren Patronenhülse aus diesem ausgezogen wird.

Alle oben beschriebenen Elemente, die in unmittelbare Berührung mit dem Patronengurt 500 gelangen, sind vorzugsweise mindestens zweimal in Längsrichtung der Waffe nebeneinanderliegend angeordnet, um sicherzustellen, daß die Patronen 502, 504, 506 während des gesamten Zubringebetriebes stets parallel zur Längsmitte 114 ausgerichtet sind und bleiben.

Beiderseits der für den Gurteinlauf benutzten Einführöffnung 118 befindet sich, wie in Fig. 2 gezeigt, eine um eine aufrechte Achse drehbar gelagerte Patronengurt-Führungsrolle 378, deren Durchmesser etwa dem einer Patrone 502, 504, 506 entspricht. Hierdurch wird ein sauberer Gurteinlauf gewährleistet.

Diese Patronengurt-Führungsrollen 378 können auch an der anderen Einführöffnung 116 angebracht werden.

### Die Abzugseinrichtungsgruppe 400:

Die Abzugseinrichtungsgruppe weist die eigentliche Abzugseinrichtung 402 auf, die in einem gehäuseartigen Kasten untergebracht ist, der an der Rückseite der Endabdeckung 110 des Gehäuses 102 angebracht ist und zwischen den beiden Handgriffen 146, 148 sitzt.

Beiderseits des Kastens ist in ergonomischer Zuordnung zu den Handgriffen 146, 148 jeweils eine Daumenplatte 406 angebracht, die als Abzug dient und mit dem Abzugshebel 404 so verbunden ist, daß sich beim Niederdrücken einer oder beider der Daumenplatten 406 der Abzugshebel 404 mit seinem freien Ende hebt, dabei den Abzugsstollen 230 freigibt und somit den Masseverschluß 202 nach vorne schnellen läßt.

Unterhalb der Daumenplatte sitzt an einer oder jeder Seitenwand des Kastens ein Sicherungs- und Feuerwahlhebel, der drehfest auf einer Welle 408 (Fig. 10) angebracht ist.

Der Sicherungs- und Feuerwahlhebel hat ebenso wie die Welle 408 drei Drehlagen: S (Sicher), E (Einzelfeuer) und D (Dauerfeuer). Die in Fig. 10 gezeigte Stellung ist die Drehlage S (Sicher).

Der Aufbau der zugeordneten Sicherungs- und Feuerwahleinrichtung ist herkömmlich und hier nicht gezeigt; in der Drehlage S sind die Daumenplatten 406 und der Abzugshebel 404 arretiert, in den anderen Drehlagen freigegeben; zusätzlich wird in der Drehlage E nach einmaligem Schwenken des Abzugshebels 404 die Verbindung zwischen diesem und den Daumenplatten 406 unterbrochen, so daß der Abzugshebel 404 nach Auslösung eines Schusses seine Abzugsstollen-Haltelage wieder einnehmen kann, auch wenn die Daumenplatten 406 niedergedrückt bleiben; in der Drehlage D sind Daumenplatten 406 und Abzugshebel 404 ständig fest bewegungsverbunden.

Zusätzlich zur beschriebenen, bekannten Sicherungseinrichtung hat aber die Welle 408 zusätzlich einen unrunden, in Fig. 10 gezeigten Steuerabschnitt, der vom gegabelten Ende des einen Schenkels (Abstützschenkels) 418 eines Sicherungs-Winkelhebels 424 umgriffen ist.

In der gezeigten Sicherungslage S wird der gegabelte Abstützschenkel 418 mit seiner hinteren Endkante gegen einen Anschlag 420 angedrückt. In der Einzelfeuer- und Dauerfeuerlage E und D wird dagegen der Abstützschenkel 418 durch den unrunden Steuerabschnitt der Welle 408 vom Anschlag 420 wegbewegt.

Der Sicherungs-Winkelhebel 424 ist im Bereich seines Scheitels schwenkgelagert und weist als zweiten Schenkel einen Fanghaken 412 auf, der sich in der Sicherungslage S nach vorne bis über den Fangvorsprung 232 des Masseverschlusses 202 hinaus erstreckt und diesen umgreift.

Auf dem Sicherungs-Winkelhebel sitzt ferner ein Nocken 410, der eine Abflachung aufweist, die in der gezeigten Sicherungslage S flächig gegen eine Druckplatte 422 anliegt, die ihrerseits schwenkbar gelagert ist und von einer Feder gegen den Nocken 410 hin belastet ist.

Wie in Fig. 10 gezeigt, haben der Fangvorsprung 232 und das freie Ende des Fanghakens 412 eine komplementäre Ausbildung, so daß sie sich fest gegenseitig hintergreifen und miteinander verhaken können, wenn der Masseverschluß 202 etwa infolge eines Bruchs des Abzugshebels 404 trotz der Wahl der Sicherungslage S beginnt, sich nach vorne zu bewegen.

Im Gegensatz zur Drehlage S ist in den Drehlagen E und D der Welle 408 der Sicherungs-Winkelhebel 424 so verschwenkt, daß das hakenartig gekrümmte freie Ende des Fanghakens 412 aus der Bewegungsbahn des Fangvorsprungs 232 herausgehoben ist und die freie Bewegung des Masseverschlusses 202 nicht behindert.

Bricht allerdings der Abstützschenkel 418, so daß der Sicherungs-Winkelhebel nicht mehr auf die Drehlage der Welle 408 anspricht, dann bringt die Druckplatte 422 den Nocken 410 und damit den Fanghaken 412 in die gezeigte Sicherungslage S.

Infolge der Formgebung der Hakenanordnung ist bei deren Eingriff der Fanghaken 412 festgelegt und damit die Welle 408 blockiert, so daß es nicht möglich ist, die Waffe zu entsichern und somit ungewollt gleichzeitig abzufeuern.

Die voranstehend beschriebene, eigentliche Abzugseinrichtung löst den Masseverschluß 202 aus, nicht aber den eigentlichen Zündvorgang. Dieser wird von der in Fig. 9 schematisch dargestellten Zündeinrichtung ausgelöst, und zwar in Zuordnung zur präzisen Lage des Masseverschlusses 202; es wurde weiter oben bereits darauf hingewiesen, daß bei der erfindungsgemäßen Waffe das Einhalten eines genau definierten Zündzeitpunktes innerhalb engster Toleranzen besonders wichtig ist.

Wie bereits bei der Beschreibung der Gehäusegruppe 100 erläutert, erstreckt sich längs der Bewegungsbahn des Verschlußkopfes 224 am rechten Gehäusesteg 122 eine Steuerkurve 138 für die Schlagbolzenhülse 416 und am linken Gehäusesteg 124 eine Steuerkurve 140 für den Schlagbolzen 414.

Die Schlagbolzenhülse 416 weist einen stabförmigen, vorderen Teil und einen kolbenartigen, hinteren Teil auf, die in der mit einem entsprechenden Durchmesser bemessenen Axialbohrung 212 des Verschlußkopfes 224 hin- und herbeweglich aufgenommen ist.

Beide Teile sind von einer Hülsen-Längsbohrung 426 durchsetzt, mit einem vorderen, engen Durchlaß für die Schlagbolzenspitze, einem Hauptabschnitt für den Schlagbolzenschaft und einem erweiterten Endabschnitt zur Aufnahme des verdickten Schlagbolzenendes.

Am Außenumfang des erweiterten Endabschnitts befindet sich eine als Führungshebel-Aufnahme 428 ausgebildete Vertiefung.

Der Schlagbolzen 414 weist, wie bereits angedeutet, eine Schlagbolzenspitze, einen schlanken, mit Führungs-Ringvorsprüngen versehenen Schlagbolzenschaft und ein verdicktes Schlagbolzenende mit einer nach hinten offenen Sackbohrung auf, die zur Aufnahme einer Schlagfeder (nicht gezeigt) ausgebildet ist.

An der Außenseite des verdickten Schlagbolzenendes ist an diesem ein durchbohrter Quervorsprung ausgebildet; die zu ihrem Ende hin konisch erweiterte Bohrung des Quervorsprungs bildet eine Spannhebel-Aufnahme 434.

Das hintere Ende der Axialbohrung 212 im Verschlußkopf 224 ist von einer Federabstützbüchse 444 verschlossen, auf deren Boden sich die in der Sackbohrung im verdickten Schlagbolzenende aufgenommene Schlagfeder abstützt.

Der Verschlußkopf 224 ist von oben her bis zu seiner Axialbohrung 212 an den Stellen, an denen die Bewegungsbereiche der Führungshebel-Aufnahme 428 und der Spannhebel-Aufnahme 434 liegen, geschlitzt; innerhalb der so gebildeten Schlitzanordnung liegen hintereinander drei an einer jeweils zugehörigen, horizontalen Querachse im Verschlußkopf 224 gelagerte Steuerelemente.

Das vorderste dieser Steuerelemente ist ein Führungshebel 430, der wie eine Wiege geformt ist und mit seinen beiden vorspringenden Enden an der Steuerkurve 138 für die Schlagbolzenhülse 416 entlangläuft.

Wie ersichtlich, ist die Kipplage des Führungshebels 430 abhängig von der Formgebung der Steuerkurve 138.

Der Führungshebel 430 weist einen rechtwinklig abstehenden, fest angebrachten Mitnahmefinger 432 auf, dessen kugelig verdicktes freies Ende in der Führungshebel-Aufnahme 428 sitzt.

Die Kipplage des Führungshebels 430 bestimmt somit zwangsweise die axiale Lage der Schlagbolzenhülse 416.

Die Steuerkurve 138 ist so ausgebildet, daß der Führungshebel 430 nur in jenem Bereich der Verschlußbewegung seine vordere Lage einnehmen kann, in dem auch die Zündung erfolgen soll. Da aber der von Teilen der Axialbohrung 212 und der Hülsen-Längsbohrung 426 gebildete Durchlaß für die Spitze des Schlagbolzens 414 nur dann kurz genug ist, um die Schlagbolzenspitze in einer für die Zündung ausreichenden Länge hindurchtreten zu lassen, wenn sich die Schlagbolzenhülse 416 in ihrer vorderen Lage befindet, ist nur in dem vorgeschriebenen engen Bereich der Verschlußbewegung, in dem die Zündung erfolgen muß, eine solche Zündung überhaupt möglich.

Das mittlere Steuerelement ist ein Spannhebel 436, der wie der Führungshebel 430 wiegenartig ausgebildet ist und an der Steuerkurve 140 entlangläuft, die seine Kipplage um seine Lagerung erzwingt.

Im Gegensatz zum Führungshebel 430 ist am vorderen, an der Steuerkurve 140 ablaufenden Ende des Spannhebels 436 eine Rolle angebracht, die die beim Spannen der Schlagfeder aufzubringenden Kräfte überträgt.

Das hintere Ende des Spannhebels 436 ist durch eine Rastvertiefung ausgespart, die der Drehachse des dritten Steuerelements (das weiter unten beschrieben wird) zugewandt ist.

Der Spannhebel weist einen etwa rechtwinklig abstehenden, fest angebrachten Spannfinger 440 auf, mit einem kugeligen freien Ende, das in der Spannfinger-Aufnahme 434 sitzt.

Angesichts der hohen, beim Spannen der Schlagfeder zu übertragenden Kräfte sind der Spannfinger 440 und die Spannfinger-Aufnahme 434 größer bemessen als der Mitnahmefinger 432 und die Mitnahmefinger-Aufnahme 428.

Das dritte, hinterste Steuerelement ist ein Auslöser 442, der als zweiarmiger Winkelhebel ausgebildet ist, dessen einer (hinterer) Arm gegen die Steuerkurve 140 oder eine eigene Steuerkurve angedrückt wird und auf dieser abläuft; der andere (vordere) Arm weist an seinem freien Ende eine Rastnase 438 auf, die bei gespannter Schlagfeder in die Rastvertiefung an der Rückseite des Spannhebels 436 einfällt.

Wie ersichtlich, kann die Steuerkurve 140 eine Kippbewegung des Auslösers 442 veranlassen, dessen Rastnase dann aus der Rastvertiefung herausgeschwenkt wird, woraufhin der Spannhebel freigegeben wird und die Schlagfeder abschlagen kann, vorausgesetzt, die örtliche Ausbildung der Steuerkurve 140 läßt dies zu.

In Fig. 9 ist die Position der Zündeinrichtung gezeigt, die sie ganz kurz vor der Zündung einnimmt, also am vorderen Ende der Steuerkurven 138, 140.

Der Führungshebel 430 hat bereits die Kipplage eingenommen, in welcher er die Schlagbolzenhülse 416 in ihre vorderste Lage versetzt hat. Der Spannhebel ist bereits über die vorderste Abschrägung der Steuerkurve 140, die sein Kippen zum Spannen der Schlagfeder veranlaßt, nach vorne hinweggelaufen, liegt aber nicht gegen diese Steuerkurve 140 an, da er vom Auslöser 442 über den Eingriff Rastvertiefung/Rastnase in seiner Lage gehalten wird. Wenn dieser Auslöser, was unmittelbar bevorsteht, von der Steuerkurve 140 nach hinten gekippt wird, dann kann der Spannhebel kippen, die Schlagfeder kann sich entspannen und der Schlagbolzen kann nach vorne schnellen und die Patrone zünden.

## Patentansprüche

1. Selbstlade-Granatwerfer mit
- einer Patronengurt-Zuführeinrichtung, die mittels in den Patronengurt eingreifender Klinken (352, 354, 358) eine Patrone bevorzugt in horizontaler Richtung bis vor ein Patronenlager fördert, wobei die Klinken von zwei in einem aufklappbaren Zubringerdeckel angeordneten, gegenläufigen, quer zur Schußrichtung beweglichen Schiebern getragen sind und nach unten abstehen,
- einem Masseverschluß, der aus einer Auslöselage durch die Federkraft einer, vorzugsweise zweier Schließfedern nach vorne längs eines Bewegungsweges gegen das Patronenlager läuft und dazu eingerichtet ist, bei diesem Vorlauf die von den Klinken geförderte Patrone aus dem Patronengurt in das Patronenlager zu schieben sowie nach deren Zündung durch den entstehenden Rückstoß den Bewegungsweg in einem Rücklauf zurückzulegen und dabei die Schließfeder(n) zu spannen,
- einer mit dem Masseverschluß und den Schiebern verbundenen Steuerung, die den Vor- und Rücklauf des Masseverschlusses in die hierzu quergerichtete Wechselbewegung der Schieber umsetzt,
- einer Zündeinrichtung mit einem Schlagbolzen, der - vorzugsweise durch eine Schlagfeder - gespannt und in gespanntem Zustand durch eine Arretierung gehalten ist, wobei die Arretierung gelöst und die Patrone gezündet wird, bevor der Masseverschluß seinen Vorlauf beendet hat, aber erst, nachdem die Patrone weit genug in das Patronenlager eingeführt ist, um dem Gasdruck des Abschusses standzuhalten, und
- einem mit dem Patronenlager verbundenen, sich längs des Bewegungsweges erstreckenden und diesen mindestens teilweise umschließenden Gehäuse, dessen Längsmitte die Mittelachse des Patronenlagers aufnimmt,
**dadurch gekennzeichnet, daß** die mit dem Masseverschluß (202) und den Schiebern (342, 344) verbundene Steuerung die folgenden Merkmale aufweist:
- ein sich längs des Bewegungsweges erstreckender, gelenkig am Gehäuse (102) gelagerter Kurvenhebel (302), der eine Steuerkurve (304) aufweist,
- hierzu komplementär am Masseverschluß (202) einen Mitnehmer (222) , so daß der Kurvenhebel (302) beim Vor- und Rücklauf des Masseverschlusses (202) hierzu quergerichtete Schwenkbewegungen durchführt, und
- ein den Kurvenhebel (302) mit den Schiebern (342, 344) verbindendes Gestänge (310, 326, 328), welches die Schwenkbewegungen in die Wechselbewegungen der Schieber (342, 344) überführt.

2. Granatwerfer nach Anspruch 1, **dadurch gekennzeichnet, daß**
- die gelenkige Lagerung (306) des etwa mittig über dem Masseverschluß (202) angeordneten Kurvenhebels (302) an dessen vorderem Ende sowie über der Längsmitte (114) des Gehäuses (102) und im Bereich der Hinterkante des Zubringerdeckels (318) erfolgt,
- der Kurvenhebel (302) etwa an der Vorderseite des hintersten Drittels seiner Länge mit dem hinteren Ende des hinteren Armes eines seitlich neben dem Kurvenhebel (302) schwenkbar am Gehäuse (102, 124) angeordneten, zweiarmigen Umlenkhebels (310) gekoppelt ist, und
- der Umlenkhebel (310) am Ende seines vorderen Armes ein lösbares Gelenkverbindungselement, vorzugsweise ein Aufnahmemaul oder einen nach oben abstehenden Umlenkzapfen (316) aufweist, der bei geöffnetem Zubringerdeckel (318) freiliegt und bei geschlossenem Zubringerdeckel (318) in ein lösbares Gelenkverbindungs-Gegenelement, bevorzugt einen Umlenkzapfen oder ein Aufnahmemaul (336), an einer Hebelsteuerung (326, 328) zum Steuern der beiden Schieber (342, 344) eingreift.

3. Granatwerfer nach Anspruch 2, **dadurch gekennzeichnet, daß** die Hebelsteuerung einen ersten Steuerhebel (326) und zu diesem zur Längsmitte symmetrisch gegenläufig beweglich einen zweiten Steuerhebel (328) aufweist.

4. Granatwerfer nach Anspruch 3, **dadurch gekennzeichnet, daß**
- der erste und der zweite Steuerhebel (326, 328) mit gleichem Abstand beiderseits der Längsmitte (114) im Zubringerdeckel (318) nahe dessen Hinterkante gelagert sind, sich um den jeweils etwa gleichen Abstand nach vorne und jeweils von der Längsmitte (114) weg erstrecken sowie mit ihrem vorderen Ende mit dem ersten bzw. dem zweiten Schieber (342, 344) gelenkig verbunden sind, und
- einer der Steuerhebel (326) vorzugsweise geradlinig über seine Lagerstelle (324) hinaus verlängert ist und im Gelenkverbindungs-Gegenelement (336) endet.

5. Granatwerfer nach Anspruch 4, **dadurch gekennzeichnet, daß**
- beide Steuerhebel (326, 328) jeweils einen bevorzugt etwa rechtwinklig von ihrer Lagerstelle (324, 322) aus aufeinander zu abstehenden Schenkel (330) aufweisen, und
- die beiden Schenkel (330) gelenkig miteinander verbunden sind.

6. Granatwerfer nach Anspruch 5, **dadurch gekennzeichnet, daß** zur gelenkigen Verbindung am freien Ende eines der beiden Schenkel (330) eine bevorzugt als Langloch (332) ausgebildete Kulisse angeordnet ist, in die ein am freien Ende des anderen Schenkels (330) angeordneter, bevorzugt als Eingriffszapfen (334) ausgebildeter Mitnehmer unter Bildung einer im wesentlichen totgangfreien Zwangssteuerung eingreift.

7. Granatwerfer nach einem der Ansprüche 3 bis 6, **dadurch gekennzeichnet, daß** die gelenkige Verbindung zwischen einerseits dem ersten oder zweiten Schieber (342, 344) und andererseits dem ersten oder zweiten Steuerhebel (326, 328) jeweils gleichartig ausgebildet'ist und vorzugsweise aus einem zum Steuerhebel (326, 328) hin abstehenden, festen Zapfen (346, 348) oder einer Rolle am Schieber (342, 344) besteht, der bzw. die in ein Langloch oder Aufnahmemaul (338) am Ende des Steuerhebels (326, 328) eingreift.

8. Granatwerfer nach Anspruch 7, bei dem jeder der beiden Schieber in einer Querführung hin- und herbeweglich geführt ist, **dadurch gekennzeichnet, daß** die beiden Querführungen (340) so übereinstimmend ausgebildet sind, daß die beiden Schieber (342, 344) austauschbar sind.

9. Granatwerfer nach einem der Ansprüche 1 bis 8, **dadurch gekennzeichnet, daß**
- in Auslöselage des Masseverschlusses (202) beide Schieber (342, 344) jeweils ihre äußerste Endlage einnehmen, in denen sie gegenüber der Längsmitte (114) am weitesten nach außen versetzt sind, wobei
-- der erste, auf der Seite des einlaufenden Patronengurtes (500) gelegene Schieber (342) zwischen seiner Aussenseite und der Längsmitte (114) eine gelenkig und abgefedert gelagerte Innenklinke (354) aufweist, die so ausgebildet ist, daß sie die erste Patrone (502) des Patronengurtes (500) in dessen Förderrichtung hintergreift und verschiebt, in Gegenrichtung jedoch über die nachfolgende Patrone (504) hinwegschwenkt, und
-- der zweite, vom Einlauf des Patronengurtes (500) abgewandte Schieber (344) auf seiner Außenseite eine feste Stütze (360) und auf seiner gegenüberliegenden Seite eine gelenkig und abgefedert gelagerte Schwenkklinke (358) ähnlich der Innenklinke (354) des ersten Schiebers (342) trägt,
- während des Masseverschluß-Vorlaufes beide Schieber (342, 344) sich gegenläufig quer zur Längsmitte (114) bewegen, wobei die Innenklinke (354) die erste Patrone (502) bis vor das Patronenlager (108) nachführt und die Stütze (360) auf der der Innenklinke (354) gegenüberliegenden Seite einen Anschlag für die Patrone (502) bildet,
- der Masseverschluß (202) in seinem letzten Vorlauf-Abschnitt die vor dem Patronenlager (108) befindliche Patrone (502) samt dem sie umgreifenden Gurtglied (508) aus der Gurtverbindung (510, 512) heraus- und die Patrone in das Patronenlager (108) hineinschiebt,
- beim öffnen des Masseverschlusses (202) die Innenklinke (354) über die nachfolgende Patrone (504) hinwegläuft, die Schwenkklinke (358) sich von der Außenseite her gegen diese Patrone (504) annähert und die Stütze (360) sich nach außen von der ausgezogenen Patronenhülse entfernt, um deren Ausziehen nicht zu behindern, und
- während des des restlichen Masseverschluß-Rücklaufes die Schwenkklinke (358) die nachfolgende Patrone (504) bis in die Lage nachführt, die sie als nunmehr erste Patrone (502) in der Auslöselage des Masseverschlusses (202) einnimmt.

10. Granatwerfer nach Anspruch 9, **dadurch gekennzeichnet, daß** der erste Schieber (342) an seiner Innenseite einen festen Anschlag (356) ähnlich der Stütze (360) aufweist, die das unerwünschte Nachrutschen der letzten Patrone eines Patronengurtes (500) verhindert.

11. Granatwerfer nach einem der Ansprüche 9 oder 10, **dadurch gekennzeichnet, daß**
- an der Außenseite des ersten Schiebers (342) eine der Innenklinke (354) ähnliche, gelenkig und abgefedert gelagerte Außenklinke (352) angeordnet ist, die beim letzten Abschnitt des Vorlaufes des Masseverschlusses (202) in ihrer wirksamen Lage verriegelt ist, so daß sie bei der Bewegung entgegen der Förderrichtung des Patronengurtes (500) nicht über die nachfolgende Patrone (504) hinwegschwenkt, und
- daß der erste Schieber (342) mit der verriegelten Außenklinke (352) eine Rückwärts- bzw. Auswärtsbewegung durchführt, in welcher die nachfolgende Patrone (504) durch den Eingriff mit der Aussenflanke der Außenklinke (352) von der bereits ausgegurteten ersten Patrone (502) entgegen der Förderrichtung wegbewegt wird, bis diese nachfolgende Patrone (504) gegen die Innenflanke der Schwenkklinke (358) anschlägt.

12. Granatwerfer nach Anspruch 11, **dadurch gekennzeichnet, daß** der zweite Schieber (344) beim letzten Abschnitt des Masseverschluß-Vorlaufes die Außenklinke (352) übergreift und sie dabei verriegelt.

13. Granatwerfer nach einem der Ansprüche 9 bis 12, **dadurch gekennzeichnet, daß** mindestens eine der Klinken (352, 354, 358), die Stütze (360) und/oder der Anschlag (356) von mindestens und bevorzugt zwei parallel zur Längsmitte (114) hintereinanderliegenden, im wesentlichen gleichartigen Bau- bzw. Funktionsteilen gebildet ist.

14. Granatwerfer nach einem der Ansprüche 9 bis 13, **dadurch gekennzeichnet, daß**
- unter dem zugeführten Patronengurt (500) auf dessen vom Zubringerdeckel (318) abgewandten Seite ein gefedert gegen den Patronengurt (500) schwenkbarer Sperrhebel (362) angeordnet ist, der dem Patronengurt (500) bei dessen Bewegung in Förderrichtung nach unten ausweicht, aber bei dessen Rückwärtsbewegung zum stützenden Eingriff in eine Patrone (502) eingerichtet ist, und
- daß er dann, wenn sich der Masseverschluß (202) in seiner Auslöselage befindet, gegen die vorderste Patrone (502) von außen her anliegt und somit die Anlage zum Einlegen des Patronengurtes (500) bildet.

15. Granatwerfer nach einem der Ansprüche 1 bis 14, mit einer an der Einführungsöffnung für den Patronengurt angeordneten Gurtführung, **dadurch gekennzeichnet, daß** die Gurtführung eine am Gehäuse (102) lösbar anbringbare Gurtführungsbühne (376) aufweist, die den Patronengurt (500) von unten her abstützt.

16. Granatwerfer nach Anspruch 15, **dadurch gekennzeichnet, daß** die Gurtführung eine Abdeckung aufweist, die mit Abstand über der Gurtführungsbühne (376) angeordnet ist und den Patronengurt (500) von oben her abdeckt und führt.

17. Granatwerfer nach Anspruch 16, **dadurch gekennzeichnet, daß** die Gurtführung beiderseits der Gurtführungsbühne (376) jeweils eine um eine vertikale Achse drehbare Patronengurt-Führungsrolle (378) aufweist.

18. Granatwerfer nach einem der Ansprüche 15 bis 17, **dadurch gekennzeichnet, daß** beiderseits des Gehäuses (102) jeweils eine Einführöffnung (116, 118) für den Patronengurt (500) vorgesehen ist, und daß die Teile der Gurtführung (376, 378) einzeln, gruppenweise oder als Gesamtheit wahlweise an einer der beiden Einführöffnungen (116, 118) anbringbar sind.

19. Granatwerfer nach Anspruch 18, **dadurch gekennzeichnet, daß** die nicht der Gurtführung (376, 378) zugeordnete Einführöffnung (116) mit einer abnehmbaren, vorzugsweise als Blechplatte (350) ausgebildeten Wandung verschließbar ist.

20. Granatwerfer nach einem der Ansprüche 18 oder 19, **dadurch gekennzeichnet, daß** beiderseits des Gehäuses (102) jeweils eine Halterung für die lösbare Befestigung eines Patronenkastens seitlich neben der jeweiligen Einführöffnung (116, 118) angeordnet ist.

21. Granatwerfer nach den Ansprüchen 16 und 20, **dadurch gekennzeichnet, daß** die Abdeckung am Patronenkasten angeordnet ist.

22. Granatwerfer nach Anspruch 21, **dadurch gekennzeichnet, daß** der Patronenkasten einen obenliegenden Deckel aufweist, der um ein Scharnier schwenkbar ist, das an der vom Granatwerfer abgewandten Seitenwand des Patronenkastens angeordnet ist, und daß die Abdeckung am Deckel befestigt oder einstückig mit diesem ausgebildet ist.

23. Granatwerfer nach einem der Ansprüche 1 bis 9, **dadurch gekennzeichnet, daß**
- unterhalb des Patronenlagers (108) ein sich parallel zum Zubringerdeckel (318) und längs des Patronengurt-Förderweges erstreckender Führungstisch (364) angeordnet ist,
- am mittleren Abschnitt des Führungstisches (364) eine Patronenauflage (366) ausgebildet ist, die hinter dem Patronenlager (108) liegt, und
- die Patronenauflage (366) dazu eingerichtet ist, mindestens bei dem zum Einführen einer Patrone (502) in das Patronenlager (108) erforderlichen Abschnitt des Masseverschluß-Vorlaufes nach unten auszuweichen, so daß die Stirnseite (208) des Masseverschlusses (202) zusammen mit den radial überstehenden Ausbildungen (514) an der Patrone (502) über den Führungstisch (364) unbehindert hinweglaufen kann.

24. Granatwerfer nach Anspruch 23, **dadurch gekennzeichnet, daß** die Patronenauflage (366) an einer ihrer Seitenkanten um eine zur Längsmitte parallele Achse schwenkbar ausgebildet und um einen über diese hinausragenden, vorzugsweise abwärts gekröpften Führungshebel (368) verlängert ist, dessen freies Ende einen Mitnehmer (370) trägt, der mit einer Gegenausbildung am Masseverschluß (202) in Eingriff bringbar ist, um das Abwärtsschwenken der Patronenauflage (366) zu steuern.

25. Granatwerfer nach Anspruch 24, **dadurch gekennzeichnet, daß** unter der von der Achse abgewandten Seitenkante der Patronenauflage (366) ein diese arretierender, schwenkbarer Klemmhebel (372) angeordnet ist, der einen Mitnehmer (374) trägt, der mit einer Gegenausbildung am Masseverschluß (202) in Eingriff bringbar ist, um die Arretierung zu lösen.

26. Granatwerfer nach einem der Ansprüche 23 bis 25, **dadurch gekennzeichnet, daß** die Patronenauflage einen sich quer zur Längsmittelebene erstreckenden Patronenauflagefinger (366) aufweist.

27. Granatwerfer nach Anspruch 26, **dadurch gekennzeichnet, daß** mindestens und bevorzugt zwei zueinander parallele, in Richtung der Längsmitte nebeneinanderliegende Patronenauflagefinger (366) angeordnet sind.

## Claims

1. A self-loading grenade launcher with
- a cartridge belt feed device, which by means of pawls (352, 354, 358) engaging in the cartridge belt conveys a cartridge preferably in the horizonal direction until it is in front of a cartridge chamber (108), wherein the pawls are borne by two counteracting slides disposed in a hinged carrier cover and movable transversely to the firing direction, and project downwards,
- an inertia bolt, which from a release position runs forward along a path under the spring force of one, preferably two return springs towards the cartridge chamber and is set up to slide the cartridge conveyed by the pawls, during this forward movement, out of the cartridge belt into the cartridge chamber and also after its firing by the resultant blowback to run back along the path in a recoil movement and in so doing to tension the return spring(s),
- a control system connected to the inertia bolt and the slides, which converts the forward and recoil movement of the inertia bolt into the alternating movement of the slides directed transversely thereto,
- a firing device with a firing pin which is tensioned - preferably by a main spring - and is held by a detent in the tensioned state, whereby the detent is released and the cartridge is fired before the inertia bolt has completed its forward travel, but only after the cartridge has penetrated far enough into the cartridge chamber to withstand the gas pressure of the discharge, and
- a housing connected to cartridge chamber, extending along the path of movement and at least partially surrounding it, the longitudinal centre of which houses the central axis of the cartridge chamber,
**characterised in that** the control unit connected to the inertia bolt (202) and the slides (342, 344) has the following features:
- a cam lever (302) which extends along the path of motion and is articulated on the housing (102), and which comprises a cam (304),
- complementary thereto on the inertia bolt (202), a catch (222) so that during the forward movement and recoil of the inertia bolt (202) the cam lever (302) performs swivel movements transversally directed to said inertia bolt, and
- a rod assembly (310, 326, 328) which connects the cam lever (302) with the slides (342, 344), and which transfers the swivel movements into the alternating movements of the slides (342, 344).

2. A grenade launcher according to Claim 1,
**characterised in that**
- the articulated bearing (306) of the cam lever (302) disposed roughly centrally above the inertia bolt (202) takes place at its front end and also above the longitudinal centre (114) of the housing (102) and in the region of the rear edge of the carrier cover (318),
- roughly at the front side of the rearmost third of its length the cam lever (302) is coupled with the rear end of the rear arm of a two-armed reversing lever (310) that is pivoted on the housing (102, 124) laterally next to the cam lever (302), and
- at the end of its front arm the reversing lever (310) comprises a detachable azticulated connecting element, preferably an opening or an upwardly protruding reversing journal (316), which is uncovered when the carrier cover (318) is open and when the carrier cover (318) is closed engages in a detachable counter-element of the articulated connection, preferably a reversing journal or an opening (336), on a lever control device (326, 328) for controlling the two slides.

3. A grenade launcher according to Claim 2,
**characterised in that** the lever control device comprises a first control lever (326) and symmetrically thereto to the longitudinal centre a second control lever (328) which moves in the opposite direction.

4. A grenade launcher according to Claim 3,
**characterised in that**
- the first and the second control levers (326, 328) are mounted with equal spacing on either side of the longitudinal centre (114) in the carrier cover (318) close to is rear edge, extend forwards by roughly the same distance in each case and in each case away from the longitudinal centre (114) and also are hinged by their front end to the first or the second slide (342, 344), and
- one of the control levers (326) is preferably extended in a straight line beyond its bearing (324) and ends in the counter-element (336) of the articulated connection.

5. A grenade launcher according to Claim 4,
**characterised in that**
- both control levers (326, 328) each have an arm (330) which preferably sticks out roughly at right angles from its bearing (324, 322) towards one another, and
- the two arms (330) are hinged to one another.

6. A grenade launcher according to Claim 5,
**characterised in that** for the articulated connection at the free end of one of the two arms (330) a crank preferably designed as a slot (332) is provided, into which a catch disposed at the free end of the other arm (330) and preferably designed as an engagement journal (334) engages whilst forming a substantially backlash-free forced control.

7. A grenade launcher according to one of Claims 3 to 6,
**characterized in that** the articulated connection between firstly the first or second slide (342, 344) and secondly the first or second control lever (326, 328) has a similar construction in each case and preferably consists of a fixed journal (346, 348) sticking out towards the control lever (326, 328) or a roller on the slide (342, 344), which engages in a slot or opening (338) at the end of the control lever (326, 328).

8. A grenade launcher according to Claim 7, in which each of the two slides is guided to move back and forward in a transverse guide,
**characterised in that** the two transverse guides (340) are constructed to match so that the two slides (342, 344) can be exchanged.

9. A grenade launcher according to one of Claims 1 to 8,
**characterised in that**
- in the release position of the inertia bolt (202) poth slides (342, 344) in each case assume their farthest final position, in which they are moved outwards to the furthest extent, wherein
-- the first slide (342) positioned on the side of the entering cartridge belt (500) between its outer side and the longitudinal centre (114) comprises a hinged and spring-mounted inner pawl (354), which is designed so that it engages behind the first cartridge (502) of the cartridge belt (500) in its direction of transport and displaces it, but in the opposite direction swivels away over the following cartridge (504), and
-- the second slide (344) remote from the inlet of the cartridge belt (500) bears a fixed support (360) on its outside and on its opposite side bears a hinged and spring-mounted swivel pawl (358) similar to the inner pawl (354) of the first slide (342).
- during the forward movement of the inertia bolt, both slides (342, 344) move in opposite directions at right angles to the longitudinal centre (114), whereby the inner pawl (354) follows the first cartridge (502) to in front of the cartridge chamber (108) and the support (360) on the side opposite the inner pawl (354) forms a stop for the cartridge (502),
- in its last stage of the forward movement, the inertia bolt (202) pushes the cartridge (502) situated in front of the cartridge -chamber (108) together with the belt member (508) encompassing it out of the belt connection (510, 512) and pushes the cartridge into the cartridge chamber (108),
- upon opening the inertia bolt (202) the inner pawl (354) runs over the following cartridge (504), the swivel pawl (358) approaches this cartridge (504) from the outside and the support (360) moves outwardly away from the extracted cartridge case so as not to impede its extraction, and
- during the remaining recoil of the inertia bolt the swivel pawl (358) follows the following cartridge (504) into the position which it now assumes as the first cartridge (502) in the release position of the inertia bolt (202).

10. A grenade launcher according to Claim 9,
**characterised in that** the first slide (342) has a fixed stop (356) on its inner side similar to the support (360), which prevents the undesired slipping down of the last cartridge in a cartridge belt (500).

11. A grenade launcher according to one of Claims 9 or 10,
**characterised in that**
- disposed on the outside of the first slide (342) is an outer pawl (352), similar to the inner pawl (354), which is hinged and spring-mounted, and which during the last stage of the forward movement of the inertia bolt (202) is locked in its active position so that it does not swivel over the following cartridge (504) during the movement against the direction of transport of the cartridge belt (500),
**and in that**
- the first slide (342) with the locked outer pawl (352) performs a backwards and outwards movement in which the following cartridge (504) is moved against the direction of transport away from the already unloaded first cartridge (502) by engagement with the outer flank of the outer pawl (352), until this following cartridge (504) strikes against the inner flank of the swivel pawl (358).

12. A grenade launcher according to Claim 11,
**characterised in that** the second slide (344) engages over the outer pawl (352) during the last stage of the forward movement of the inertia bolt and in so doing locks it.

13. A grenade launcher according to one of Claims 9 to 12,
**characterised in that** at least one of the pawls (352, 354, 358), the support (360) and/or the stop (356) is formed by at least and preferably two components and operating parts, which lie one behind the other parallel to the longitudinal centre (114) and are essentially similar.

14. A grenade launcher according to one of Claims 9 to 13,
**characterised in that**
- disposed beneath the supplied cartridge belt (500) on its side remote from the carrier cover (318) is a locking lever (362) which can swivel spring-loaded against the cartridge belt (500), and which deflects the cartridge belt (500) downwards during its movement in the direction of transport, but during its backward movement is designed for supporting engagement in a cartridge (502).
**and in that**
- when the inertia bolt (202) is in its release position, it abuts against the foremost cartridge (502) from outside and thus forms the device for the insertion of the cartridge belt (500).

15. A grenade launcher according to one of Clams 1 to 14, having a belt guide disposed at the feed opening for the cartridge belt,
**characterised in that** the belt guide comprises a belt guide stage (376) detachably mountable on the housing (102), which supports the cartridge belt (500) from below.

16. A grenade launcher according to Claim 15,
**characterised in that** the belt guide comprises a cover which is disposed at a distance above the belt guide stage (376) and covers and guides the cartridge belt (500) from above.

17. A grenade launcher according to Claim 16,
**characterised in that** on either side of the belt guide stage (376) the belt guide in each case has a cartridge belt guide roller (378) which can rotate around a vertical axis.

18. A grenade launcher according to one of Claims 15 to 17,
**characterised in that** a feed opening (116, 118) for the cartridge belt (500) is provided in each case on either side of the housing (102),
**and in that** the parts of the belt guide (376, 378) can alternatively be mounted individually, in groups or as a unit at one of the two feed openings (116, 118).

19. A grenade launcher according to Claim 18,
**characterised in that** the feed opening (116) not associated with the belt guide (376, 378) can be closed by a removable wall, preferably constructed as a sheet metal plate (350).

20. A grenade launcher according to one of Claims 18 or 19,
**characterised in that** on either side of the housing (102) a mounting for the detachable fixing of a cartridge box is provided in each Case at the side next to the respective feed opening (116, 118).

21. A grenade launcher according to Claims 16 and 20,
**characterised in that** the cover is disposed on the cartridge box.

22. A grenade launcher according to Claim 21,
**characterised in that** the cartridge box comprises a lid at the top, which can swivel around a hinge which is disposed on the side wall of the cartridge box remote from the grenade launcher,
**and in that** the cover is fixed to the lid or is constructed in one piece therewith.

23. A grenade launcher according to one of Claims 1 to 9,
**characterised in that**
- a guide table (364) that extends parallel to the carrier cover (318) and along the conveying path of the cartridge belt is disposed beneath the cartridge chamber (108),
- a cartridge support (366), which lies behind the cartridge case (108), is constructed on the central portion of the guide table (364), and
- the cartridge support (366) is designed to deflect downwards at least during the stage of the forward movement of the inertia bolt required for the introduction of a cartridge (502) into the cartridge chamber (108), so that the front side (208) of the inertia bolt (202) together with the radially projecting formations (514) on the cartridge (502) can run unimpeded over the guide table (364).

24. A grenade launcher according to Claim 23,
**characterised in that** the cartridge support (366) is constructed to pivot on one of its side edges around an axis parallel to the longitudinal centre and is extended by a downwardly cranked guide lever (368) that projects over said cartridge support, the free end of which bears a catch (370), which can be brought into engagement with a counter-element on the inertia bolt (202), so as to control the downward swivelling of the cartridge support (366).

25. A grenade launcher according to Claim 24,
**characterised in that** disposed beneath the side edge of the cartridge support (366) remote from the axis is a swivelling clamping lever (372) which stops said Cartridge support, and which bears a catch (374), which can be brought into engagement with a counter-element on the inertia bolt (202) so as to release the stopping device.

26. A grenade launcher according to Claim 26,
**characterised in that** the cartridge support has a cartridge support finger (366) which extends at right angles to the longitudinal centre plane.

27. A grenade launcher according to Claim 26,
**characterised in that** at least and preferably two mutually parallel cartridge support fingers (366) that lie next to one another in the direction of the longitudinal centre are provided.

## Revendications

1. Lance-grenades à chargement automatique comportant
- un dispositif d'introduction de la bande de cartouches, qui, au moyen de cliquets (352, 354, 358) venant en prise dans la bande de cartouches, transporte une cartouche de préférence dans une direction horizontale jusqu'en avant d'un magasin à cartouche, et dans lequel les deux cliquets sont portés par deux coulisseaux, qui sont disposés dans un couvercle d'amenée rabattable pouvant être ouvert par rabattement, se déplacent en des sens opposés, sont mobiles transversalement par rapport à la direction de tir, et font saillie vers l'arrière,
- une culasse, qui, à partir d'une position de déclenchement, circule vers l'avant le long d'un trajet de déplacement en direction du magasin à cartouche, sous l'action de la force d'un ressort et de préférence de deux ressorts de fermeture et est agencée de manière à repousser, lors de son déplacement d'avance, la cartouche entraînée par les cliquets à partir de la bande de cartouches pour l'introduire dans le magasin à cartouche, et pour, lors de l'amorçage de cette cartouche, parcourir le trajet de déplacement dans un mouvement de recul sous l'effet du recul et bander le ou les ressorts de fermeture,
- un organe de commande, qui est relié à la culasse et aux coulisseaux et qui convertit le déplacement d'avance et le déplacement de recul de la culasse en le déplacement alternatif des coulisseaux, qui est orienté transversalement par rapport aux mouvements de la culasse,
- un dispositif de mise à feu comportant un percuteur qui est armé - de préférence au moyen d'un ressort d'armement
- et est retenu à l'état armé par un dispositif d'arrêt, par un verrou, le verrou étant libéré et la cartouche mise à feu avant que la culasse ait terminée son déplacement d'avance, mais uniquement après que la cartouche a été introduite suffisamment loin dans le magasin à cartouche, afin de résister à la pression des gaz de la mise à feu, et
- un carter, qui est relié au magasin à cartouche, s'étend le long du trajet de déplacement et entoure au moins partiellement ce magasin à cartouche et dont le centre longitudinal est situé sur l'axe médian du magasin à cartouche,
**caractérisé en ce que** l'organe de commande, qui est relié à la culasse (202) et aux coulisseaux (342, 344), présente les caractéristiques suivantes:
- un levier à came (302), qui s'étend le long du trajet de déplacement, est monté de manière articulée sur le carter (102) et comporte une came de commande (304),
- un organe d'entraînement (222) prévu d'une manière complémentaire à cela, sur la culasse (220) de telle sorte que le levier à came (302) exécute, lors du déplacement d'avance et de recul de la culasse (209), des mouvements de pivotement dirigés transversalement par rapport à ce déplacement, et
- une tringlerie (310, 326, 328), qui relie le levier à câble (302) aux coulisseaux (342, 344) et convertit les mouvements de pivotement en les déplacements alternatifs des coulisseaux (342, 344).

2. Lance-grenades selon la revendication 1, **caractérisé en ce que**
- le soutien articulé (306) du levier à came (302) disposé approximativement au centre au-dessus de la culasse (202), s'effectue sur son extrémité avant ainsi qu'au moyen de l'axe longitudinal (114) du carter (102) et dans la zone du bord arrière du couvercle d'amenée (318);
- le levier à came (302) est couplé, approximativement au niveau du côté avant du tiers le plus en arrière de sa longueur, à l'extrémité arrière du bras arrière d'un levier de renvoi à deux bras (310), qui est disposé latéralement à côté du levier à came (320) de manière à pouvoir pivoter sur le boîtier (102, 124), et
- le levier de renvoi (310) comporte, sur l'extrémité de son bras avant, un élément de liaison articulé détachable, de préférence une embouchure de réception ou un embout de déviation (316) qui fait saillie vers le haut et qui, lorsque le couvercle d'amenée (318) est ouvert est libre et, lorsque le couvercle d'amenée (318) est fermé, s'engage dans un élément antagoniste amovible de liaison articulé, de préférence un embout de déviation ou une mâchoire de réception (336), avec un organe de commande à levier (326, 328) pour la commande des deux coulisseaux (342, 344).

3. Lance-grenades selon la revendication 2, **caractérisé en ce que** l'organe de commande à levier comporte un premier levier de commande (326) et possède un second levier de commande (328) qui est déplaçable en sens opposé du levier précédent et ce symétriquement par rapport à l'axe longitudinal.

4. Lance-grenades selon la revendication 3, **caractérisé en ce que**
- les premier et second leviers de commande (326, 328) peuvent être montés, à la même distance des deux côtés de l'axe longitudinal (114) dans le couvercle d'amenée (318), à proximité du bord arrière de ce dernier, de manière à s'étendre à peu près sur la même distance vers l'avant et respectivement à partir du l'axe longitudinal (114), et sont reliés de façon articulée, par leur extrémité avant, au premier ou au second coulisseau (342, 344), et
- l'un des leviers de commande (326) se prolonge de préférence avec une forme rectiligne au-delà de son point de support (324) et se termine dans l'élément antagoniste (336) de la liaison articulée.

5. Lance-grenades selon la revendication 4, **caractérisé en ce que**
- les deux leviers de commande (324, 326) possèdent chacun des branches respectives (330) qui font saillie respectivement à angle droit à partir de leurs points de support (324, 326), en direction l'une de l'autre, et
- les branches (330) sont reliées entre elles de façon articulée.

6. Lance-grenades selon la revendication 5, **caractérisé en ce que** pour la liaison articulée au niveau de l'extrémité libre de l'une des deux branches (330), il est prévu une coulisse, qui est agencée de préférence sous la forme d'un trou allongé (332) et dans laquelle un organe d'entraînement, qui est disposé sur l'extrémité libre de l'autre branche (330) et est agencé de préférence sous la forme d'un téton d'engrènement (334), s'engage en formant une unité de commande forcée essentiellement sans course à vide.

7. Lance-grenades selon l'une des revendications 3 à 6, **caractérisé en ce que** la liaison articulée est formée respectivement de la même manière entre d'une part le premier ou le second coulisseau (342, 344) et d'autre part le premier ou le second levier de commande (326, 328), et est constituée de préférence par un embout fixe (346, 348), qui fait saillie à partir du levier de commande (326, 328), ou par un rouleau situé sur le coulisseau (342, 344), ce téton ou ce rouleau s'engageant dans un trou allongé ou une mâchoire de réception (338), situé sur l'extrémité du levier de commande (326, 328).

8. Lance-grenades selon la revendication 7, dans lequel chacun des deux coulisseaux est guidé de manière à être déplaçable en va-et-vient dans un guide transversal, **caractérisé en ce que** les deux guides transversaux (340) sont agencés de manière identique de sorte que les deux coulisseaux (342, 344) sont permutables.

9. Lance-grenades selon l'une des revendications 1 à 8, **caractérisé en ce que**
- dans la position de déclenchement de la culasse (202), les deux coulisseaux (342, 344) occupent respectivement leurs positions d'extrémité les plus extérieures, dans lesquelles ils sont décalés au maximum vers l'extérieur par rapport à l'axe longitudinal (114), et
- le premier coulisseau (342), qui est installé sur le côté de la bande entrante de cartouches (500), comporte, entre son côté extérieur et l'axe longitudinal (114), un cliquet intérieur (354), qui est monté de façon articulée et en étant suspendu élastiquement et est agencé de telle sorte qu'il s'engage derrière la première cartouche (502) de la bande de cartouches (500) et la déplace dans la direction d'entraînement de cette bande de cartouches, mais pivote en sens opposé au-dessus de la cartouche suivante (504),
- le second coulisseau (344), qui est tourné à l'opposé de l'entrée de la bande de cartouche (500), porte, sur son côté extérieur, un appui fixe (360) et, sur son côté opposé, un cliquet pivotant (358) monté de manière articulée et en étant suspendu élastiquement, et qui est analogue au cliquet intérieur (354) du premier coulisseau (342),
- pendant le déplacement d'avance de la culasse, les deux coulisseaux (342, 344) se déplacent en des sens opposés transversalement par rapport à l'axe longitudinal (114), le cliquet intérieur (354) suivant la première cartouche (502) jusqu'en avant du magasin à cartouche (108) et l'appui (360) formant, sur le côté situé à l'opposé du cliquet intérieur (354), une butée pour la cartouche (502),
- lors de sa dernière phase d'avance, la culasse (202) dégage la cartouche (502) située en avant du magasin à cartouche (108), ainsi que l'élément de bande (508), qui l'enserre, hors de la liaison de bande (510, 512) et repousse la cartouche dans le magasin à cartouche (108),
- lors de l'ouverture de la culasse (202), le cliquet intérieur (354) circule au-dessus de la cartouche suivante (504), le cliquet pivotant (358) se rapproche de cette cartouche (504) à partir du côté extérieur et l'appui (360) s'écarte vers l'extérieur à partir de la douille de cartouche extraite, pour ne pas gêner son extraction, et
- pendant le reste du mouvement de recul de la culasse, le cliquet pivotant (358) suit la cartouche suivante (504) jusque dans la position qu'elle prend en tant que maintenant première cartouche (502) dans la position de déclenchement de la culasse (202).

10. Lance-grenades selon la revendication 9, **caractérisé en ce que** le premier coulisseau (342) comporte, sur son côté intérieur, une butée fixe (356) similaire à l'appui (360), et qui empêche un dérapage indésirable de la dernière cartouche d'une bande de cartouches (300).

11. Lance-grenades selon la revendication 9 ou 10, **caractérisé en ce que**
- sur le côté extérieur du premier coulisseau (342) est disposé un cliquet extérieur (352) qui est semblable au cliquet intérieur (354) et est monté de manière articulée et en étant suspendu élastiquement et qui, lors de la dernière phase du mouvement d'avance de la culasse (202), est verrouillé dans sa position active de sorte que lors du déplacement, il ne pivote pas au-dessus de la cartouche suivante (504) lors du déplacement en sens opposé de la direction d'entraînement de la bande de cartouches (500), et
- que le premier coulisseau (342) exécute, avec le cliquet extérieur (352) verrouillé, un déplacement de recul ou de sortie, lors duquel la cartouche suivante (504) est écartée en sens opposé de la direction d'entraînement par la première cartouche (502) déjà dégagée de la bande, sous l'effet de l'engrènement avec le flanc extérieur du cliquet extérieur (352), jusqu'à ce que cette cartouche suivante (504) s'applique contre le flanc intérieur du cliquet pivotant (358).

12. Lance-grenades selon la revendication 11, **caractérisé en ce que** lors de la dernière phase du déplacement d'avance de la culasse, le second coulisseau (344) s'engage par dessus le cliquet extérieur (352) et le verrouille .

13. Lance-grenades selon l'une des revendications 9 à 12, **caractérisé en ce qu'**au moins l'un des cliquets (352, 354, 358), l'appui (360) et/ou la butée (356) sont formés par au moins un et de préférence deux composants ou parties fonctionnelles essentiellement identiques, qui sont situés l'un derrière l'autre parallèlement à l'axel longitudinal (114).

14. Lance-grenades selon l'une des revendications 9 à 13, **caractérisé en ce que**
- au-dessous de la bande de cartouches amenée (500), sur le côté de cette bande tourné à l'opposé du couvercle d'amenée (318), est disposé un levier de blocage (372), qui peut pivoter élastiquement contre la bande de cartouches (500), et qui écarte vers le bas la bande de cartouches (500) hors de son déplacement dans sa direction d'entraînement, mais qui est agencé, pour s'engager avec une action de soutien dans une cartouche (502) lors du déplacement de recul de la bande, et
- que lorsque la culasse (202) est située dans sa position de déclenchement, le levier s'applique contre la couche la plus avancée (502), à partir de l'extérieur, et par conséquent forme l'appui pour l'insertion de la bande de cartouches (500).

15. Lance-grenades selon l'une des revendications 1 à 14, comportant un guide-bande disposé au niveau de l'ouverture d'introduction pour la bande de cartouches, **caractérisé en ce que** le guide-bande comporte une plateforme (367) de guidage de la bande, qui peut être monté de façon amovible sur le boîtier (102) et qui supporte la bande de cartouches (500) à partir du bas.

16. Lance-grenades selon la revendication 15, **caractérisé en ce que** le guide-bande comporte un capot, qui est disposé à distance au-dessus de la plateforme (367) de guidage de la bande et recouvre à partir du haut la bande de cartouches (500) et la guide.

17. Lance-grenades selon la revendication 16, **caractérisé en ce que** le guide-bande comporte des deux côtés de la plateforme (376) de guidage de la bande, respectivement un rouleau (378) de guidage de la bande de cartouches, qui peut tourner autour d'un axe vertical.

18. Lance-grenades selon l'une des revendications 15 à 17, **caractérisé en ce que** des deux côtés du boîtier (102) il est prévu respectivement une ouverture d'introduction (116, 118) pour la bande de cartouches (500) et que les parties du guide-bande (376, 378) peuvent être montées individuellement, par groupes ou dans leur totalité au choix au niveau de l'une des deux ouvertures d'introduction (116, 118).

19. Lance-grenades selon la revendication 18, **caractérisé en ce que** l'ouverture d'introduction (116), qui n'est pas associée au guide de bande (376, 378), peut être fermée par une paroi amovible, qui est agencée de préférence sous la forme d'une plaque en tôle (350).

20. Lance-grenades selon l'une des revendications 18 ou 19, **caractérisé en ce que** des deux côtés du boîtier (102) est prévu respectivement un dispositif de retenue pour la fixation amovible d'une boîte de cartouches, et ce latéralement d'un côté de l'ouverture respective d'introduction (116, 118).

21. Lance-grenades selon les revendications 16 et 20, **caractérisé en ce que** le capot est monté sur la boîte de cartouches.

22. Lance-grenades selon la revendication 21, **caractérisé en ce que** la boîte de cartouches comporte un couvercle supérieur, qui peut pivoter autour d'une charnière et est disposé sur la paroi latérale de la boîte de cartouches, qui est tournée à l'opposé du lance-grenades, et que le capot est fixé sur le couvercle ou est réalisé d'un seul tenant avec ce dernier.

23. Lance-grenades selon l'une des revendications 1 à 9, **caractérisé en ce que**
- au-dessous du magasin à cartouche (108) est disposée une table de guidage (364), qui s'étend parallèlement au couvercle d'amenée (318) et le long du trajet d'entraînement de la cartouche,
- dans la section médiane de la table de guidage (364) est formé un appui pour cartouche (366), qui est situé en arrière du magasin à cartouche (108), et
- l'appui pour cartouche (366) est agencé de manière à s'écarter vers le bas au moins lors de la phase du mouvement d'avance de la culasse, qui est nécessaire pour l'introduction d'une cartouche (502) dans le magasin à cartouche (108), de telle sorte que la face frontale (208) de la culasse (202) peut, conjointement avec les configurations (151), qui font saillie radialement sur la cartouche (502), circuler librement au-dessus de la table de guidage (364).

24. Lance-grenades selon la revendication 23, **caractérisé en ce que** l'appui pour cartouche (366) est agencé de manière à pouvoir pivoter sur l'un de ses bords latéraux, autour d'un axe parallèle à l'axe longitudinal et se prolonge par un levier de guidage (368) qui fait saillie au-delà de ce bord et est de préférence coudé vers l'aval et dont l'extrémité libre porte un organe d'entraînement (370), qui peut être amené en prise avec une configuration antagoniste prévue sur la culasse (202), de manière à commander le pivotement vers l'aval de l'appui pour cartouche (366).

25. Lance-grenades selon la revendication 24, **caractérisé en ce que** au-dessus du bord latéral, éloigné de l'axe, de l'appui pour cartouche (366) est disposé un levier de serrage pivotant (372), qui bloque cet appui et qui porte un organe d'entraînement (364), qui peut être amené en prise avec une configuration antagoniste prévue sur la culasse (202) de manière à libérer le blocage.

26. Lance-grenades selon l'une des revendications 23 à 25, **caractérisé en ce que** l'appui pour cartouche comporte un doigt (366) qui s'étend transversalement par rapport au plan médian longitudinal.

27. Lance-grenades selon la revendication 26, **caractérisé en ce qu'**il est prévu au moins un et de préférence deux doigts (366) d'appui de cartouche, qui sont parallèles entre eux et sont disposés côte-à-côte dans la direction de l'axe longitudinal.
